(19) Europäisches Patentamt
European Patent Office
Office européen des brevets

(11) **EP 2 830 503 B1**

(12) **EUROPEAN PATENT SPECIFICATION**

(45) Date of publication and mention of the grant of the patent:
**27.12.2017 Bulletin 2017/52**

(51) Int Cl.:
***A61B 6/00*** *(2006.01)*    ***A61B 5/02*** *(2006.01)*

(21) Application number: **13769655.5**

(22) Date of filing: **26.03.2013**

(86) International application number:
**PCT/CN2013/073205**

(87) International publication number:
**WO 2013/143444 (03.10.2013 Gazette 2013/40)**

(54) **METHOD AND APPARATUS FOR OBTAINING VASODILATION DATA REPRESENTING CUTANEOUS LOCAL THERMAL HYPEREMIA RESPONSE OF A SUBJECT**

VERFAHREN UND VORRICHTUNG ZUR GEWINNUNG VON VASODILATATIONSDATEN ZUR DARSTELLUNG DES ANSPRECHENS EINES PATIENTEN AUF EINE KUTANE LOKALE THERMISCHE HYPERÄMIE

MÉTHODE ET APPAREIL D'OBTENTION DE DONNÉES RELATIVES À LA VASODILATATION REPRÉSENTANT UNE RÉPONSE D'HYPERÉMIE THERMIQUE LOCALE CUTANÉE D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2012 US 201261615865 P**

(43) Date of publication of application:
**04.02.2015 Bulletin 2015/06**

(73) Proprietor: **New Chinese Biotechnology Corporation Ltd.**
**Taipei City 100 (TW)**

(72) Inventors:
• **HUANG, Chung-Shin**
**Taipei City 100**
**Taiwan (TW)**
• **TSAI, Yuan-Feen**
**Taipei City 106**
**Taiwan (TW)**
• **WANG, Shwu-Fen**
**New Taipei City 235**
**Taiwan (TW)**

(74) Representative: **Lang, Christian**
**LangPatent Anwaltskanzlei**
**IP Law Firm**
**Rosenheimer Straße 139**
**81671 München (DE)**

(56) References cited:
CN-A- 1 806 757        US-A- 4 859 078
US-A1- 2004 067 270        US-A1- 2011 172 749

**Description**

**BACKGROUND OF THE INVENTION**

1. FIELD OF THE INVENTION

The present disclosure relates to a method for obtaining a value of at least one vasodilation parameter representing cutaneous local thermal hyperemia response of a subject.

2. DESCRIPTION OF RELATED ART

Prior studies have indicated that assessment of the microvasculature in an accessible body area may not only delineate the status of the local microvasculature at the sampling site, but it also correlates well with various disorders/diseases associated with vascular and neurological dysfunctions throughout the body. Cutaneous microcirculation situated 1 to 2 mm below the skin surface is often referred to as skin blood flow (SkBF), and multiple techniques for assessing skin blood flow have been developed.

Laser Doppler techniques, among others, are commonly used to investigate skin blood flow. Laser Doppler is based on the reflection of a beam of laser light. Light undergoes changes in wavelength (Doppler shift) when it hits moving blood cells. The magnitude and frequency distribution of these changes in wavelength are related to the number and velocity of blood cells. Currently available laser Doppler techniques include, but are not limited to, laser Doppler flowmetry (LDF).

LDF technique uses a probe located on the skin to record velocities and concentrations of moving blood cells in a small volume of 1 mm$^3$ or smaller, depending on the incident wavelength. Several different signals can be recorded but the red blood cell flux (RBCF, i.e. the product of the velocity and concentration of moving blood cells within the measuring volume) is used most often. Local heating of the skin causes a direct and substantial vasodilation in the area being heated which is known as local thermal hyperemia (LTH). In healthy humans, a sustained local temperature of about 42-44°C causes maximal dilation of skin blood vessels. LDF combined with LTH is a non-invasive method for evaluating microvascular reactivity and neural and endothelial functions; therefore, LTH assessed by LDF has been used to study peripheral vascular disease, chronic renal failure, and diabetes. Furthermore, a diminished thermal hyperemia response has been found in various diseases, such as systemic sclerosis, spinal cord injury, peripheral neuropathy, and multiple system atrophy.

Axon reflex in human skin has been suggested to be an afferent C-fiber-mediated response to mechanical, heating, or chemical stimulation. Axon reflex induces antidromic vasodilatation via the release of calcitonin gene-related peptide and substance P. Sensory nerve block by local anesthetics reduces the quick heating-induced AR response by about 60%, indicating that the contribution of sensory nerve to the AR flare is higher than other factors. Impairments in axon reflex-related vasodilatation can affect the microcirculation of the skin in diabetic and other small-fiber neuropathy. Axon reflex is impaired in peripheral neuropathy, in which smaller nerve fibers are affected earlier than larger fibers. Because neuropathy occurs earlier than vasculopathy in peripheral neuropathy-related diseases, the test involved integrated endothelial function may be not as sensitive as the test with neural-mediated portion of endothelial function. For example, initial peak induced by axon reflex is neural-mediated, while maximal dilatation or initial peak expressed as percentage of maximal dilatation is associated with an integrated neural- and vascular-mediated endothelial function. Therefore, short-heating LTH, which focuses on the neural-mediated portion of LTH, becomes a potential objective and non-invasive tool for sensory testing.

Dysfunction of sympathetic activity is very common in many psychological, neurological, and general medical diseases. The quantitative evaluation of sympathetic function is essential to assess disease status and treatment outcome. Except examination of generalized sympathetic functions, microneurography is the only technique that can directly record sympathetic nerve activity in muscle tissue, but this invasive method is not commonly applied because it is highly technique-dependent.

Axon reflex (AR) enhancement has been demonstrated by norepinephrine and other α-adrenergic agonist iontophoresis. The presynaptic blockade of sympathetic vasoconstrictor nerves abolished the AR response to local skin heating. Therefore, testing the local AR skin response has the potential to be an objective method for assessing the challenge effects of acute chemical or physical intervention on local sympathetic function and studying the factors involved in sympathetic activity in healthy and diseased conditions.

The skin blood flow change during LTH comprises two major independent phases, an initial maximum (i.e., the initial phase) followed by a nadir, then a prolonged plateau (i.e., the plateau phase). The initial maximum usually takes place during the first 5 minutes after the local heating and has been proven to be primarily mediated by axon reflex via antidromic activation of afferent C-fiber, and be affected by nitric oxide and sympathetic vasoconstrictor nerves. By contrast, the plateau occurs not until 20 to 30 minutes after the local heating and is mediated largely by nitric oxide (NO) that can induce a direct relaxation of smooth muscles within the vascular wall. Maximal dilatation of blood vessel is achieved by continuous heating for at least 35 min. Most conventional practices use data associated with the plateau phase (or maximal dilatation) to assess the microvascular condition of the subject; however, this is very time consuming. Moreover, the prolonged heating required to obtaining plateau data results in the over-vasodilation of the blood vessels, and it generally takes more than 2 hours for the blood vessels to recover to their original or pre-heated conditions. Prior studies have indicated that repeated application of a local thermal stimulus on the same sampling site causes a time-dependent reduction in the vasodilatory response to this stimulus. This effect is associated with a temporary decrease in the local sensitivity of skin microcirculation to the dilating action of NO. Accordingly, all studies in this field suggest that a repeated sampling shall not be taken until blood vessels have recovered to their original conditions or after the test subject has undergone the recovery period; otherwise the obtained data would not reflect the actual microvascular condition of the subject and is useless due to the desensitization of the blood vessels. Furthermore, the previously used assessments of the LTH response represent the integrated effects of both neural- and endothelial-mediated function and not merely neural-mediated effects; therefore, they can't be used to assess local sensory and sympathetic function.

In addition, single-point probe LDF has been considered poorly reproducible for LTH by previous protocol. This is partly due to the minimal recording area and random recording sites in conjunction with the great spatial heterogeneity in capillary density and highly variable in sensory innervation. In view of the foregoing, enormous variation in the laser Doppler flux are found from comparable sites between subjects, from different sites in the same subject, and even from the same site in the same individual at intervals of minutes, hours, or days. Therefore, this technique can't be used in the routine assessment of microvascular and neurological function and alternative methods with larger recording area like LDI, LSCI, and integrated probe are introduced, however, they are still time-consuming and other limitations arise. Because the holder size of an integrated probe combined with a heating probe or LDI (also LSCI) heating probe is markedly larger than the holder size of a single-point probe combined with a heating probe, they are difficult to apply to many locations on the body, such as fingers, toes and uneven regions of the face and joints, further limiting their use.

Therefore, there is continued interest in the development of new methods for non-invasively obtaining the value of vasodilation parameter representing cutaneous local thermal hyperemia response of a subject with satisfactory data reproducibility in a time-efficient manner. Of particular interest would be the development of such methods and apparatus, in which a repeated sampling may be performed in a relatively short interval as compared with convention LDF/LTH techniques.

CN 1 806 757 A discloses a method for three-dimensional imaging of skin angiopathy and an intelligent treatment system. The system makes use of a Doppler/coherent signal processing system and of a laser device, employing the selective absorption of infrared laser by indocyanine green.

US 4 859 078 A relates to an apparatus for the non-invasive measurement of thermal properties and perfusion rates of biomaterials by means of at least two heating and temperature sensors. By suitable arrangement of the sensors, a net heat flow between the first sensor and the medium can be measured, allowing determination of thermal conductivity, thermal diffusivity and/or fluid perfusion within the medium.

US 2004/067270 A1 relates to pharmaceutical preparations for the treatment of itch, nausea, hyperalgesia and the complications of opioid agonists. The preparations suppress the activation of sensory afferent nerves.

US 2011/0172749 A1 discloses methods and apparatus for enhancing vascular access in an appendage to enhance therapeutic and interventional procedures, i.e. for increasing blood flow, controlling the vasodilatation of a patient's vascular structure.

## SUMMARY

The following presents a simplified summary of the disclosure in order to provide a basic understanding to the reader. This summary is not an extensive
overview of the disclosure and it does not identify key/critical elements of the present invention or delineate the scope of the present invention. Its sole purpose is to present some concepts disclosed herein in a simplified form as a prelude to the more detailed description that is presented later.

In one aspect, the present disclosure is directed to a method for obtaining a value of at least one vasodilation parameter representing the cutaneous local thermal hyperemia response of a subject. In particular, said cutaneous local thermal hyperemia response is related to the subject's neural function (such as sensory function and sympathetic activity) that is generally resulted from short term heating. Therefore, short-term heating and corresponding evaluation step provided herein are advantageous over long-term heating in evaluating neural function of a subject. Moreover, in clinical medicine, it is often required to obtain information associated with sensory function and sympathetic activity. However, the long-term heating employed by the conventional methods usually results in a desensitizing effect on microvascular response in a later sampling step. Thus, the present method is advantageous in that it is possible to minimize this desensitizing effect by using information associated with the initial phase which only requires a short-term heating.

According to embodiments of the present method, the value of the vasodilation parameter could be calculated from measurements obtained in an initial heating period. For example, the initial heating period may comprise the first 3 to 5 minutes after the local temperature reaches 38-44°C. In this way, the present sampling step would not result in sustained vasodilation (or over-vasodilation), and hence a repeated sampling could be performed at an interval of less than 2 hours. Therefore, the present method is quite time-efficient as compared with conventional LDF/LTH techniques. Further, the thus-obtained initial maximum cutaneous vascular conductance (CVC) exhibits acceptable reproducibility and is suitable for use as a factor in assessing/evaluating the cutaneous local thermal hyperemia response of the subject.

According to one embodiment of the present disclosure, the method comprises the steps as follows. Performing a baseline measurement at a first sampling site at a first temperature. During the period of baseline measurement, a first baseline red blood cell flux ($RBCF_{BL}$) of the sampling site and a first baseline mean arterial pressure ($P_{BL}$) of the subject are obtained. A first baseline cutaneous vascular conductance ($CVC_{BL}$) is calculated by the formula of: $CVC_{BL}$ = $RBCF_{BL}/P_{BL}$. After the baseline measurement, the temperature of the first sampling site of the subject is raised from the first temperature to a second temperature, and the second temperature is maintained for an initial heating period of about 2 to 14 minutes. During the initial heating period, a plurality of first initial RBCFs ($RBCF_{I, 1-n}$) of the first sampling site at a plurality of time points ($T_{1-n}$) are recorded to identify a first initial maximum RBCF ($RBCF_{I, max}$) of the sampling site. Meanwhile, a first initial mean arterial pressure ($P_I$) of the subject is also recorded. Thereafter, a first initial maximum cutaneous vascular conductance ($CVC_{I,max}$) is calculated by the formula of: $CVC_{I,max}$= $RBCF_{I, max}/P_I$. According to certain embodiments of the present disclosure, the first $CVC_{I, max}$ is used as the vasodilation parameter. Alternatively, the vasodilation parameter may be derived from the $CVC_{I, max}$ by appropriate calculation.

In one optional embodiment, the vasodilation parameter derived from the first $CVC_{I, max}$ is a first initial maximum CVC change ($\Delta CVC$) which is obtained by the formula of: $\Delta CVC = CVC_{I, max} - CVC_{BL}$

In another optional embodiment, an initial area-under-the-curve (initial AUC), another vasodilation parameter derived from the first $CVC_{I, max}$, is calculated to elucidate the accumulation of blood flux during the first 3-5 minutes of the heating period. Specifically, the $RBCF_{I, 1-n}$ are plotted against $T_{1-n}$ to obtain a curve having a function of F(X), and the initial AUC is calculated by the formula of: $AUC = \int_0^t F(X)dX$, where t is equal to or greater than the time at which the $RBCF_{I, max}$ is measured.

In optional embodiments, the first initial maximum CVC ($CVC_{I, max}$), the first initial maximum CVC change ($\Delta CVC$), or the initial AUC is then used, alone or in combination, to evaluate the cutaneous local thermal hyperemia response of the subject at a specific site of the body.

According to optional embodiments of the present disclosure, the baseline measurement is performed for about 2 to 5 minutes. However, the present disclosure is not limited thereto.

The present method is advantageous in that it is mainly based on the vascular-related measurements obtained during the initial heating period. In certain embodiments, the initial heating period may be less than 3 minutes. For example, if the sampling site is heated to the desired temperature with a greater heating rate, the $RBCF_{I, max}$ may be recorded at 2 minutes. In other embodiments, the initial heating period is 3 to 10 minutes; preferably 3 to 5 minutes. However, the present invention is not limited thereto, and the initial heating period could be maintained as according to the operator's desire; yet the measurements obtained during the first 10 minutes are preferred, more preferably the first 5 minutes, in the calculation of the vasodilation parameter(s). In certain embodiments, an algorithm involving the $CVC_{I, max}$, as well as one or more vascular-related measurements obtained during a later time of the initial heating period or one or more subsequent heating step following or immediately following the initial heating period, may be used in the calculation of the present vasodilation parameter. In other embodiments, the method of this invention does not require additional, subsequent heating after the initial heating step so as to prevent the sustained vasodilation of the sampling site, and hence a repeated sampling, if desired, could be performed at an interval of less than 2 hours.

Further, embodiments of the present aspect are advantageous in that they are time-efficient and reproducible. Accordingly, for a single subject, measurements/parameter values obtained from different sampling sites at the same time or different times, as well as measurements/parameter values obtained from the same sampling site at different times, are also comparable to one another. Moreover, measurements/parameters obtained from different subjects are also comparable to one another.

According to embodiments of the present disclosure, the vascular-related measurements, including, the first baseline RBCF, the first initial RBCFs and the first initial maximum RBCF, are measured using a laser Doppler-based probe having a diameter of less than 5 mm.

In optional embodiments, the first temperature is about 28-35°C; and the second temperature is about 38-44°C. Still optionally, the temperature is raised at a heating rate of 0.02-0.2°C/sec. In various embodiments of the present invention, the heat may be applied directly or remotely to the sampling site.

In another aspect, the present disclosure is directed to a laser Doppler-based apparatus for implementing the method according to the above-mentioned aspect(s)/embodiment(s).

In one embodiment, the measuring system includes a user interface, a laser Doppler detector, a heating unit, a controller, a computing unit, a database and a comparison unit. The user interface is configured to receive an input from a user and provide an output to the user. The laser Doppler detector is configured to measure at least one vascular-related measurement at a sampling site of the subject during a first sampling procedure. The vascular-related measurement is any of: a first baseline red blood cell flux ($RBCF_{BL}$) or a first baseline mean arterial pressure ($P_{BL}$) obtained during a baseline measurement, or a first initial RBCF ($RBCF_{I, 1-n}$), a first initial maximum RBCF ($RBCF_{I, max}$) or a first initial mean arterial pressure ($P_I$) obtained during an initial heating period of about 2 to 14 minutes. The heating unit is configured to heat the sampling site, and the controller is configured to control the laser Doppler detector and the heating unit based on the user input or a default setting. The computing unit is configured to calculate the value of the vasodilation parameter from the at least one vascular-related measurement, wherein the vasodilation parameter is or is derived from a first initial maximum cutaneous vascular conductance ($CVC_{I, max}$), calculated by the formula of: $CVC_{I, max} = RBCF_{I, max} / P_I$. The database comprises a normative value of the vasodilation parameter obtained from a population of healthy subjects. The comparison unit is configured to compare the vasodilation value of the subject with the normative value.

In optional embodiments, the present laser Doppler-based apparatus may further comprise an analysis unit which is configured to evaluate the sensory nerve function and/or sympathetic activity of the subject based on the comparison result from the comparison unit.

According to certain embodiments of the present disclosure, the output provided by the user interface is at least one of, the vascular-related measurement(s), the value(s) of the vasodilation parameter(s), the comparison result from the comparison unit, and the analysis result from the analysis unit.

Still optionally, the present laser Doppler-based apparatus may further comprise a storage unit which is configured to store at least one additional vascular-related measurement measured at the same sampling site or at least one different sampling site of the subject, concurrently with or after the first sampling procedure. In this case, the computing unit is further configured to calculate an additional value of the at least one vasodilation parameter from the at least one additional vascular-related measurement; the comparison unit is further configured to compare the additional value of the vasodilation parameter of the subject with the normative value and/or with the first vasodilation value of the same subject; and the analysis unit is further configured to evaluate the sensory nerve function and/or sympathetic activity of the subject and/or the subject's response to an intervention based on the comparison result from the comparison unit.

In various embodiments, the vasodilation parameter may be the first $CVC_{I, max}$. Alternatively, the vasodilation parameter may be derived from the first $CVC_{I, max}$ according to embodiments and examples disclosed herein, as well as equivalents thereof. For example, the vasodilation parameter derived from the first $CVC_{I, max}$ may be $\Delta CVC$ or initial AUC.

According to optional embodiments of the present disclosure, the laser Doppler detector comprises a probe having a diameter of less than 5 mm.

Many of the attendant features and advantages of the present disclosure will become better understood with reference to the following detailed description.

## BRIEF DESCRIPTION OF THE DRAWINGS

The present description will be better understood from the following detailed description read in light of the accompanying drawings, where:

Figure 1 is a schematic diagram illustrating the laser Doppler-based apparatus according to one embodiment of the present disclosure; and
Figure 2 is a schematic diagram illustrating sampling sites according to one working example of the present disclosure.

## DESCRIPTION

The detailed description provided below is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

The present disclosure provides a non-invasive and readily applicable method to study the cutaneous local thermal hyperemia response of a subject from at least one sampling site in at least one sampling session. In particular, the cutaneous local thermal hyperemia response is associated with the subject's sensory function and/or sympathetic activity. The present method is advantageous at least in that it is highly reproducible between different measurements, and therefore the method finds its utility in the fields of diagnosis and treatment of sensory and/or sympathetic disorders. For example, the values of a same vasodilation parameter between a subject suffering from said disorder and a health individual is substantially different, and the subsequent comparison/evaluation step using the calculated value would put the obtained values to practical real world use. Also, the present method is capable of monitoring the subject's changes in the LTH over time, and identifying his/her responses in the LTH to potentially harmful or beneficial interventions.

Embodiments of the present disclosure provide methods for obtaining a value of at least one vasodilation parameter representing the cutaneous local thermal hyperemia response of a subject. As discussed in the background, the initial maximum assessed by single-point LDF on the forearm is not reproducible with the currently available protocols. By contrast, vascular-related measurements (and as a result, values of the vasodilation parameters) obtained in accordance with methods of the present disclosure exhibit acceptable intrasession and intersession reproducibilities, and accordingly, these measurements/parameter values are suitable for monitoring changes in LTH of the subject over time. Furthermore, since the vasodilation parameter is or is derived from the first initial maximum CVC, it eliminates the need of collecting data from the plateau or maximum period as employed in conventional LDF/LTH techniques in which plateau or maximal CVC and other parameters are used for further assessment. The short heating period adopted in the present invention is also advantageous in that repeated sampling on the same site could be performed as early as 30 minutes after the first sampling, which gives the physician an opportunity to observe or identify responses in LTH that arise from any given treatment.

Microvascular dysfunctions have been observed in patients with wide variety of disorders. For example, sensory sensitization and sympathetic hyperactivity enhance the cutaneous local thermal hyperemia response, while sensory impairment and sympathetic inhibition reduce the cutaneous local thermal hyperemia response. Also, impairments in axon reflex-related vasodilatation are often observed in patients suffering from in diabetic and neuropathy, in which the microcirculation of the skin and smaller nerve fibers are affected.

Trigeminal neuralgia is a neuropathic disorder which causes hyperalgia. The sensory nerve of the subject suffering from trigeminal neuralgia is sensitized and hence the cutaneous local thermal hyperemia response at a specific site is enhanced. For post-herpetic skin, the sensory nerve at a specific site is damaged and the cutaneous local thermal hyperemia response there is reduced. The sympathetic nerve of patients suffering from complex regional pain syndrome is in a hyperactive state, and hence may enhance the cutaneous local thermal hyperemia response at a specific site.

In view of the foregoing, also included in the present disclosure are methods for evaluating/assessing the health and/or disease status of a subject. The present method provides an objective and non-invasive tool for sensory and sympathetic testing and the early evaluation of peripheral neuropathy, by focusing on the neural-mediated response of cutaneous local thermal hyperemia (or cutaneous LTH). Further, the present method is also useful in evaluating local sympathetic activity and the somatosympathetic reflex during or after physical or chemical interventions.

In one example, this is achieved by establishing multiple normative values of vasodilation parameter(s) obtained from various sampling sites of a population of healthy subjects. The vasodilation parameter may be the first initial maximum CVC or other parameters derived therefrom; e.g., the first initial maximum CVC change, and the initial AUC.

In practice, these normative values could be used as the reference values for assessing the health/disease status of the subject. It is known that the autonomic nerve dysfunction may influence the hyperemic reactivity. Therefore, initial maximum CVCs associated with multiple sampling sites could be compared with the normative values respectively associated with the sampling sites to determine whether the subject is suffering from autonomic nerve dysfunction.

In another example, the evaluating/assessing method is achieved by comparing the initial maximum CVC of the subject with a previously or later obtained initial maximum CVC to determine the progress of the treatment or disease. For example, previous investigations have indicated that cutaneous local thermal hyperemia response is impaired in subjects with diabetes; in particular, said impairment usually progresses from peripheral toward central of the body of a subject. Hence, for a subject with diabetes, a comparison between initial maximum CVCs associated with peripheral vasculature and central vasculature is useful for determining the status of the disease. It is also feasible to use these normative values as references to determine the effect of any given therapy.

The practices of the present invention are hereinafter described in detail with respect to methods for obtaining a value of at least one vasodilation parameter representing the cutaneous local thermal hyperemia response of a subject, methods for evaluating/assessing the cutaneous local thermal hyperemia response of the subject, methods for correlating the cutaneous local thermal hyperemia response to the health/disease status of the subject, and methods for evaluating/assessing the subject's response to a treatment. Results of LDF/LTH, as described hereinbelow, indicate that data obtained in accordance with the present method exhibit acceptable intersession and/or intrasession reproducibility, and as such these data are suitable for use in the evaluating/assessing and correlating step described herein.

In one aspect, the present disclosure is directed to a method for obtaining a value of at least one vasodilation parameter representing the cutaneous local thermal hyperemia response of a subject.

According to one embodiment of the present disclosure, the method comprises the following steps:

(a) performing a baseline measurement at a first sampling site of the subject at a first temperature and obtaining a first baseline red blood cell flux ($RBCF_{BL}$) of the sampling site and a first baseline mean arterial pressure ($P_{BL}$) of the subject;

(b) raising the temperature of the first sampling site from the first temperature to a second temperature;

(c) maintaining the second temperature for an initial heating period of about 2 to 14 minutes, and recording a plurality of first initial RBCFs ($RBCF_{I, 1-n}$) of the first sampling site at a plurality of time points ($T_{1-n}$) to identify a first initial maximum RBCF ($RBCF_{I, max}$) and recording a first initial mean arterial pressure (PI) of the subject during the heating period; and

(d) calculating the value of the vasodilation parameter, wherein the vasodilation parameter is or is derived from a first initial maximum cutaneous vascular conductance ($CVC_{I, max}$), calculated by the formula of: $CVC_{I, max} = RBCF_{I, max} / P_I$.

LTH in practice, the subjects are placed in a temperature controlled room ($25\pm1°C$) prior to the baseline measurement (step (a)); this step is referred to as acclimation and is designated to guard against problems associated with measuring basal flux using laser Doppler flowmetry. According to embodiments of the present disclosure, the acclimation period is about 30 to 60 minutes; preferably 60 minutes. Further, the entire heating/sampling step described herein is carried out in the same temperature-controlled condition. Optionally, the room is also humidity-controlled with 40-60% humidity.

According to embodiments of the present disclosure, the baseline measurement is performed for at least 2 minutes. For example, period for baseline measurement is about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 minutes. Preferably, the period for baseline measurement is about 2 to 5 minutes.

In one embodiment, the measurement of the red blood cell flux in the baseline and initial sampling steps (steps (a) and (c)) is carried out by a single-point probe laser Doppler flowmetry..

In step (b), the temperature of the first sampling site is raised by applying heat directly or remotely to the first sampling site. Any conventional instrument and technique suitable for providing heat to the sampling site could be used herein. For example, at least one thermoelectric heating element could be attached to the probe to provide heat thereto. In another example, a heating device such as an infrared lamp or a halogen lamp could be placed remotely over the sampling site thereby heating the sampling site. According to some embodiments of the present disclosure, the first temperature is about 28-35°C and the second temperature is about 38-44°C. Preferably, the first temperature is about 33°C and the second temperature is about 42°C in accordance with working examples provide hereinbelow. Preferably, the temperature is raised at a rate of 0.02-0.2°C/sec; preferable 0.1°C/sec.

There are no particular limitations on sampling sites as long as they are accessible for sampling in accordance with methods provided herein. A non-limiting list of examples of body parts suitable for use herein includes the forehead, face, neck, earlobe, arm, forearm, elbow, wrist, finger, chest, abdomen, back, breast, leg, knee, ankle, foot and toe. Although prior researches have indicated that laser Doppler data collected at forearm exhibit poor reproducibility due to the great spatial heterogeneity in forearm capillary density, our test results indicated otherwise, and the forearm data obtained in accordance with methods provided herein exhibit acceptable reproducibility. Therefore, in some preferred embodiments of the present invention, the first sampling site is located at one of the forearms of the subject.

According to principles and spirits of the present invention, the vasodilation parameter representing the cutaneous local thermal hyperemia response of a subject are collected during the first phase of the local thermal hyperemia response. Experimental results indicated that the initial maximum RBCF is achieved during the first five minutes of heating in accordance with the methods provided herein. Therefore, in step (c), the second temperature is maintained for 3 to 5 minutes; preferably 3 to 4 minutes, and the red blood cell flux of the sampling site during this period is monitored and recorded. However, the present disclosure is not limited thereto. For example, a shorter initial heating period could be achieved with a faster heating rate, and a longer initial heating period may be required by a slower heating rate. Specifically, the initial heating period may be 2, 3, 4, 5, 6, 7, 8, 9, 10, 11, 12, 13, or 14 minutes. Generally, the initial maximum RBCF could be calculated from the average of data within the 30 or 60 seconds around the observed peak flux. Our results, as discussed hereinbelow, indicate that there is no significant difference between the 30- and 60-second-averaged initial maximum RBCFs, and hence, the 30-second-averaged initial maximum RBCF is used in the present disclosure for the sake of discussion. In one example, the sampling site is sampled with a first probe of 32°C during the baseline measurement, and then during the initial heating period, the first probe is replaced with a second probe of 42 °C. In this case, the maximum RBCF is observed around 84.75 seconds (raw data) after the start of the initial heating period, and the 30-second-averaged initial maximum RBCF is recorded around 104.25 seconds. In addition, the arterial pressure of the subject during this period is also recorded, and a mean arterial pressure is then calculated.

In step (d), the value of at least one vasodilation parameter may be calculated. The vasodilation parameter or other parameters derived therefrom may be used to evaluate the cutaneous local thermal hyperemia response of the subject. For example, the value of the first initial maximum cutaneous vascular conductance ($CVC_{I, max}$) is calculated by dividing the first initial maximum RBCF ($RBCF_{I, max}$) by the first initial mean arterial pressure ($P_I$), As another example, the first initial maximum CVC change ($\Delta CVC$) is calculated by first dividing the first baseline RBCF ($RBCF_{BL}$) by the first baseline mean arterial pressure ($P_{BL}$) to obtain a first baseline CVC ($CVC_{BL}$), and then subtracting the first baseline CVC ($CVC_{BL}$) from the first initial maximum CVC ($CVC_{I, max}$). According to yet another example, an initial area-under-the-curve (initial AUC), in which the $RBCF_{I, 1-n}$ are plotted against $T_{1-n}$ to obtain a curve having a curve function of F(X), and the initial AUC is calculated by the formula of: $AUC = \int_0^t F(X)dX$ , where t is equal to or greater than the time at which the $RBCF_{I, max}$ is measured. For example, the time "t" may be 3, 3.5, 4, 4.5, or 5 minutes, according to certain non-limiting embodiments of the present disclosure.

It should be noted that various known parameters capable of representing the axon reflex and/or endothelial-mediated functions of a subject have been evaluated in the experiments discussed hereinabove, and the results indicate that the thus-obtained initial maximum CVC exhibits the most desirable reproducibility in representing cutaneous local thermal hyperemia responses as compared with other parameters, including those suggested by prior studies such as plateau or maximal CVC.

. The value(s) of the vasodilation parameter(s) obtained in the step (d) is/are useful in many aspects. As a non-limiting example, a comparison between the first initial maximum CVC and a reference initial maximum CVC is made, so as to identify whether there is any significant variation therebetween. As could be appreciated by persons with ordinary skills in the art, it is also feasible to establish a reference data regarding ΔCVC or initial AUC, and the evaluation may be conducted based on the comparison between the as-collected data and the reference data. It should be noted that, for the purpose of such evaluation, any information obtained during a later portion of the initial heating period or one or more subsequent heating step following or immediately following said initial heating period is not a requisite component, , according to certain embodiments of the present disclosure. Rather, such information may be optional.

Optionally, there is no subsequent heating after the steps (b) and (c). As such, the present method would not result in the sustained vasodilation of the sampling site, and hence a repeated sampling, if desired, could be performed at an interval of less than 2 hours.

According to various embodiments of the present disclosure, the reference value may be a normative value of initial maximum CVCs obtain from a population of healthy subjects or a ratio of two normative values, each associated with a different body part. Alternatively, the reference value may be least one additional initial maximum CVC obtained from the subject before, concurrently with, or after the first sampling step (step (c)). For example, if the first sampling site is located at a first body part, then the reference initial maximum CVC could be a normative value of the initial maximum CVC associated with the first body part obtained from a population of healthy subjects. In this case, the subsequent comparison/evaluation step would be to compare the first initial maximum CVC taken on the first sampling site of the test subject with the normative value of the initial maximum CVC taken on the same sampling site from the healthy population.

The present methods are also inventive in that a repeated sampling could be performed within two hours after the first sampling step is completed. The ability to perform a short-term repeated sampling with data that are not confounded with vasodilation in the plateau or maximal dilatation phase is advantageous. Therefore, some optional embodiments of the present disclosure provide methods for obtaining a second initial maximum CVC of the same subject in a repeated sampling step.

Generally, the repeated sampling step may be carried out at a time interval of less than two hours from the first sampling step. Specifically, the sampling interval between any two sampling steps may be 0.5 to 2 hours; preferably 1 hour; and more preferable 45 minutes. For example, the sampling interval may be 30 or 45 minutes, or 1, 1.5, or 2 hours.

Optionally, a physical or chemical intervention may be applied to the subject after the first sampling step is completed. These physical and chemical interventions are known to affect the cutaneous local thermal hyperemia response, and thus could be used to test or treat diseases/disorders associated with microvascular or neurological dysfunction(s). In this case, data obtained in the repeated sampling could be used to evaluate/assess the effectiveness of said intervention. Illustrative examples of the physical intervention include, but are not limited to, the application of thermal energy (heat or cryotherapy), pressure, cautery, electricity, radio frequency (RF) energy, laser, radioactivity, ultrasound, balloon inflation, physical abrasion, acupuncture, and moxibustion. Non limiting list of examples of chemical intervention include the administration of acetylcholine, cholinergic agents, anticholinergic agents, phenylephrine, adrenergic agonist, adrenergic antagonist, nicotine, nitroglycerin, phosphodiesterase inhibitor (such as sildenafil), anti-inflammatory medication (such as statin), angiotensin converting enzyme (ACE) inhibitor, prostaglandins, prostanoid antagonist, histamine, antihistamine, non-steroid anti-inflammatory drug (NSAID), and anesthetic agent.

According to embodiments of the present disclosure, the repeated sampling could be carried out at a second sampling site of the subject by performing steps (a) to (d) as described hereinabove to obtain a second initial maximum CVC, a second initial maximum CVC change (ΔCVC), or a second initial AUC associated with the second sampling site. Then, the cutaneous local thermal hyperemia response of the subject is evaluated based on at least one of the first and second initial maximum CVCs or a combination thereof. Alternatively, the evaluation may be conducted based on at least one of the first and/or second ΔCVC, or the first and/or second initial AUC. For example, a comparison between the first and second initial maximum CVCs could be made to identify whether there is any significant variation therebetween. Alternatively, the first and second initial maximum CVCs could be compared with the first and second normative values of the initial maximum CVCs associated with the first and second body parts obtained from a population of healthy subjects. Still alternatively, a specific value between the first and second initial maximum CVCs could be calculated to obtain a sampling specific value; a specific value between the first and second normative values to obtain a normative specific value; and a comparison between the sampling specific value and the normative specific value is made, so as to evaluate the cutaneous local thermal hyperemia response of the subject. The sampling specific value may be a ratio of the first and second initial maximum CVCs; and the normative specific value may be a ratio of the first and second normative values. It should be noted that, in this evaluation step, any information obtained during a subsequent heating step immediately following said period during the evaluating step is not required.

In practice, the second sampling site may locate on an area substantially the same as the first sampling site, though this is not a requisite. In some optional embodiments, the second sampling site is different from the first sampling site, but both sampling sites are located on a same body part (e.g., head, neck, trunk, and limbs). Alternatively, the two sampling sites may be anatomically symmetric points. For example, the first sampling site may locate on the left forearm, whereas the second sampling site may locate on the right forearm. Still alternatively, the first and second sampling sites are respectively located at a first body part and a second body part different from the first body part.

It is another aspect of the present disclosure to provide computer executable instructions configured to perform at least one task in connection with the method described hereinabove. Generally, computer executable instructions, such as program nodules, include routines, programs, objects, components, data structures, and/or other elements that perform particular tasks or implement particular abstract data types. Typically, the functionality of the program modules may be combined or distributed as desired in various implementations. According to embodiments of the present disclosure, the tasks to be performed include raising and maintaining the temperature of the sampling site, obtaining the initial maximum RBCF of the sampling site, calculating the initial maximum CVC associated with the sampling site, and comparing the initial maximum CVC with a reference value.

Also included in the present disclosure are laser Doppler-based apparatus for implementing the method described hereinabove.

According to various embodiments of the present disclosure, the laser Doppler-based apparatus comprises a laser Doppler device adapted to implement the present method. For example, the laser Doppler device may be a laser Doppler flowmetry (LDF)

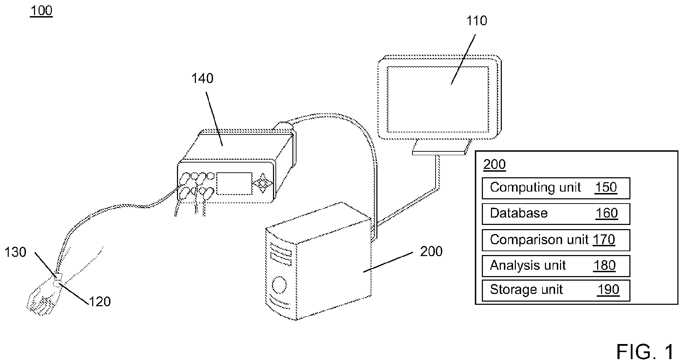

Figure 1 schematically illustrates a laser Doppler-based apparatus 100 according to one embodiment of the present disclosure. Generally, the laser Doppler-based apparatus 100 comprises a user interface 110, a laser Doppler detector 120, a heating unit 130, a controller 140, a computing unit 150, a database 160 and a comparison unit 170.

The user interface 110 is configured to receive an input from a user and/or provide an output to the user. The user interface 110 illustrated in Figure 1 is embodied as a display (or monitor). For example, the display could be a touch screen which can accept a touch input and allow a user to select certain objects displayed on the screen. However, the present invention is not limited thereto. The laser Doppler-based apparatus 100 may further comprise one or more output devices (such as a speakers, printer, or the like) and/or input devices (e.g., mouse, keyboard, or adjustment knob or button), which alone or in combination with one another may embody the user interface 110.

The laser Doppler detector 120 is configured to measure at least one vascular-related measurement at a sampling site of the subject during one or more sampling procedure. According to optional embodiments of the present disclosure, the laser Doppler detector comprises a probe, which could be a single-point probe or an integrated probe. In certain embodiments of the present disclosure, the probe may have a diameter of less than 5 mml preferably, less than 2.5 mm; more preferably, about 1.5 mm. Using probes with a smaller diameter is particularly advantageous in the case where smaller recording sites are intended to be sampled since the size of an integrated probe combined with a heating probe in general is too large to be placed on many areas of the body.

Non-limiting examples of vascular-related measurements include $RBCF_{BL}$ $P_{BL}$, $RBCF_{I, 1-n}$, $RBCF_{I, max}$ and $P_I$. In one embodiment, the vascular-related measurements recorded by the laser Doppler detector 120 are provided to the user through the user interface 110.

The heating unit 130 is configured to heat the sampling site. The heating unit 130, as illustrated in Figure 1, heats the sampling site by directly contacting the sampling site. However, the present disclosure is not limited thereto. In other embodiments, the heating unit is a remote heating means which provides heat energy to the sampling site without directly contacting thereto.

The controller 140 is configured to control the laser Doppler detector 120 and the heating unit 130 based on the user input or a default setting. It should be noted that, although the controller 140 and user interface 110 are illustrated as two physically separate components in Figure 1, the present invention is not limited thereto. For example, in certain embodiments, part or all functionalities of the user interface 110 may be embodied as one or more adjustment knob or button on the controller 140 so that the user may provide inputs to the controller by tuning the knob or button.

The computing unit 150, database 160 and comparison unit 170 are operated collectively to process the measurements obtained by the laser Doppler detector 120; the processing procedure transforms the abstract data of measurements into vasodilation-related values with meanings in the diagnosis and/or treatment senses. To facilitate the understanding of the present disclosure, a computer 200 is illustrated in Figure 1. As could be appreciated, the computer 200 is only one example of a suitable operating environment and is not intended to suggest any limitation as to the scope of use or functionality of the implementations described herein.

Specifically, the computing unit 150 is configured to calculate the value of at least one vasodilation parameter from the vascular-related measurement(s). For example, the vasodilation parameter is or is derived from a first initial maximum cutaneous vascular conductance ($CVC_{I, max}$), calculated by the formula of: $CVC_{I, max} = RBCF_{I, max} / P_I$. Further examples include the $\Delta CVC$ and initial AUC that are derived from the first $CVC_{I, max}$. In one embodiment, the value of the vasodilation parameter calculated by the computing unit 150 is provided to the user through the user interface 110.

The database 160 comprises at least one normative value. For example, the normative value may be derived from the initial maximum CVCs, $\Delta CVC$ or initial AUC obtained from a population of healthy subjects. Specifically, the normative value may be the mean or median of the population, or other statistically-obtained value that is representative of the healthy population.

The comparison unit 170 is configured to compare the value of the vasodilation parameter of the subject with the normative value and generate a comparison result. In one embodiment, the comparison result is provided to the user through the user interface 110. For example, the comparison result may indicate whether the value of the vasodilation parameter of the subject is higher than, equal to, or lower than the normative value. Also, the comparison result may indicate the extent to which the value of the vasodilation parameter of the subject is aberrant from the normative value.

In optional embodiments, the laser Doppler-based apparatus 100 may further comprise an analysis unit 180 which is configured to evaluate the sensory nerve function and/or sympathetic activity of the subject based on the comparison result from the comparison unit 110. Specifically, the analysis unit 180 may comprise informational regarding the relationship between the level of the cutaneous local thermal hyperemia response at one or more specific sampling site and a particular disorder or abnormality. Therefore, the analysis result may indicate the presence or progress of a specific disorder in a subject.

Still optionally, the present laser Doppler-based apparatus 100 may further comprise a storage unit 190. The storage unit 190 is configured to store at least one additional vascular-related measurement measured at the same sampling site or at least one different sampling site of the subject, concurrently with or after the first sampling procedure. In this case, the computing unit 150 is further configured to calculate an additional value of at least one vasodilation parameter from the at least one additional vascular-related measurement; the comparison unit is further configured to compare the additional value of the vasodilation parameter of the subject with the normative vasodilation parameter and/or with the first value of the vasodilation parameter; and the analysis unit is further configured to evaluate the sensory nerve function and/or sympathetic activity of the subject and/or the subject's response to an intervention based on the comparison result from the comparison unit.

The storage unit 190 includes volatile, nonvolatile, removable, and non-removable media implemented in any method or technology for storage of information. Examples of storage unit 190 suitable for use herein include, but are not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can accessed by the laser Doppler-based apparatus 100.

The computer 200, as illustrated in Figure 1, is a personal computer; however, the present invention is not limited thereto. In one example, the computer 200 may be physically integrated with the controller 140. Other well-known computing devices, environments, and/or configurations that may be suitable for use with such implementations include, but are not limited to, server computers, hand-held or laptop devices, personal digital assistants, smart phones, multiprocessor systems, microprocessor-based systems, programmable consumer electronics, network PCs, minicomputers, mainframe computers, distributed computing environments that include any of the above systems or devices, and the like. Computer 100 may be described in the general context of computer-executable instructions, such as program modules, executed by one or more computers or other devices. Generally, program modules include routines, programs, objects, components, data structures, and/or other elements that perform particular tasks or implement particular abstract data types. Typically, the functionality of the program modules may be combined or distributed as desired in various implementations.

The following Examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention to its fullest extent.

The primary aim of these working examples is to investigate the intrasession and intersession reproducibility of short-term heating cutaneous local thermal hyperemia assessed using single-point LDF. The secondary aim was to compare the reproducibility between different forms of data expression.

## EXAMPLES

### EXAMPLE 1: Local Thermal Hyperemia Test and Data Analysis

Healthy male and female volunteers (*n* = 20 each), aged 20-40 years, were recruited from website advertisements. The exclusion criteria included any significant neurological, cardiovascular, dermatological, and psychological history, diabetes, cigarette smoking, pregnancy, and delivery that occurred less than 1 month earlier. All female participants joined this study were at their non-menstruation period. The study was approved by the Institutional Review Board of National Taiwan University Hospital (Taipei, Taiwan, R.O.C.), and all participants gave informed written consent before participation. The participant characteristics are summarized in Table 1. No significant differences between the 30- and 60-min acclimation groups were found with regard to age, stature, body weight, and resting mean arterial pressure ($p > 0.05$).

All participants (aged $25.5 \pm 3.8$ years) were asked to be free of caffeine, tea, medications, transcutaneous electrical nerve stimulation, massage, and acupuncture on the day of the test, and were fast for at least 1 hour before the test began. Upon arrival in the laboratory, the subjects were arranged on chairs in a temperature- and humidity-controlled laboratory (40-60% humidity, $25 \pm 1°C$). They were randomly assigned to 30- and 60-min acclimation groups (*n* = 20 per group, balanced between males and females). After a 30 or 60 minutes acclimation period, the thermal hyperemia test was performed twice, 45 minutes apart (Day 1). The same test was repeated 1 to 3 days later (Day 2). The experiment was performed by two operators, and all of the procedures for the same subject were conducted by the same operator. During the study period, the outdoor temperature was approximately 12-23°C.

Table 1. Participant characteristics in the 30- and 60-min acclimation groups.

|  | 30 min acclimation (n = 20) | 60 min acclimation (n = 20) |
|---|---|---|
| Age (years) | $24.9 \pm 3.5$ | $26.0 \pm 4.1$ |
| Stature (cm) | $166.7 \pm 8.7$ | $165.9 \pm 6.8$ |
| Body weight (kg) | $59.8 \pm 12.1$ | $60.8 \pm 10.3$ |
| Mean arterial pressure (mm Hg) | $78.8 \pm 5.1$ | $77.6 \pm 6.8$ |
| Data are expressed as mean $\pm$ SD. p > 0.05 for all comparisons between two groups. | | |

The participants were supine during the entire test period. Cutaneous blood flow was measured by LDF (Moor DRT4, Moor Instruments Ltd, Axminster, Devon, UK). For each measurement, blood pressure was recorded continuously with a non-invasive blood pressure monitor (CareTaker, Empirical Technologies Corporation, Charlottesville, Virginia, USA) on the middle finger of the dominant hand throughout the procedures.

Blood flow was recorded simultaneously on the upper one-fourth of the bilateral forearm using LDF (DP12-V2 probe, 2 channels, length: 12 mm, diameter 1.5 mm; Moor Instruments Ltd.), and two recording sites were located at the radial half of the flexor and extensor aspects of the forearm, which were located according to a predefined rule. The probe was mounted to a probe holder to hold the probe in position. The extensor and flexor recording sites were located along the line between the extensor and flexor aspects of the lateral epicondyle of the humerus, respectively, and the lateral side of the styloid process of radius. The same sites were measured bilaterally with the flexor and extensor aspects in a random sequence. The recording sites were kept thermally neutral at 33°C by a heater (SH02-SHP2 probe, Moor Instruments Ltd.) for 5 minutes for the baseline measurement, and then the temperature was increased to 42°C by 1°C /10 seconds and maintained at 42°C for up to 5 minutes. After completing the measurements, the holders were maintained *in situ,* and all of the procedures were repeated with the same recording sequence 45 minutes after the first measurement. The holders were then removed without marking after the first day of the experiment. The same procedures were conducted at the same time 1 to 3 days later, and the recording sites were relocated according to the same predefined rule.

MoorSOFT package (Moor Instruments Ltd.) provided a fixed time constant value of a low pass filter, enabling the demonstration of trends of the flux signal throughout the recording. Raw flux signals were converted to Microsoft Office Excel 2007 (Microsoft Corporation, Redmond, Washington, USA) and analyzed off-line. Several built-in functions were assigned to certain cells of the spreadsheet to calculate the data for analysis.

Cutaneous red blood cell flux (RBCF), expressed as arbitrary perfusion units (PUs), was used to indicate local cutaneous blood flow, and CVC (PU/mmHg), which derived from RBCF divided by mean arterial pressure to adjust for the influence of blood pressure, was the indicator of blood flow change.

During the 5 minutes baseline measurement, two periods of baseline blood flux were evaluated, including the average of the 1.5-3.0 minutes measurement and the average of the last 2 minutes measurement before heating. Two peak CVC values were tested, including the average of 30 seconds and the average of 60 seconds around the peak flux.

Baseline CVC, peak CVC, absolute change of peak CVC, percent change of peak CVC, 4 minutes area-under-the-curve (AUC), and time to peak flux were used as indicators of the hyperemic response during heating. The absolute change of peak CVC was defined as the peak CVC minus baseline CVC, and the percentage of peak CVC change equals to the absolute change of peak CVC divided by baseline CVC multiplied by 100%. The 4 minutes AUC was the accumulation of blood flux during the first 4 minutes of the heating period. The time to peak flux was the period from the heating started and up to peak flux.

All data are expressed as mean $\pm$ SD and were analyzed using SPSS 17.0 software (SPSS Inc., Chicago, Illinois, USA). Within-subject coefficient of variation (CV) and intra-class correlation coefficient (ICC) were used to test the reproducibility of the measurements of the hyperemic response. CV values of < 10%, 10-25%, and > 25% represented good, moderate, and poor reproducibility, respectively. ICC values > 0.75, of 0.40-0.75, and < 0.40 represented excellent, good to fair, and poor agreement, respectively. The standard error of measurement (SEM) and minimal detectable change (MDC) were further assessed for the most reproducible forms of data expression. The SEM was calculated as pooled SD multiplied by the square root of (1-ICC), and MDC was calculated as SEM multiplied by the square root of 2 multiplied by 1.96 for $n$ = 20.

**EXAMPLE 2: Intrasession Reproducibility**

No significant differences were found in the CVC data between Day 1 and Day 2 or between the 30- and 60-minute acclimation groups. Therefore, only the data from the two tests on the first day in the 30 minutes acclimation group are listed and presented in different forms in Table 2.

Table 2. Cutaneous local thermal hyperemia data on the first day in the 30-min acclimation group.

| | Test | RF | RE | LF | LE |
|---|---|---|---|---|---|
| Baseline blood flux (PU) | 1st | 20.97 $\pm$ 16.36 | 20.50 $\pm$ 13.28 | 20.03 $\pm$ 14.07 | 18.64 $\pm$ 10.63 |
| | 2nd | 15.73 $\pm$ 5.47 | 17.43 $\pm$ 6.17 | 16.25 $\pm$ 7.77 | 16.05 $\pm$ 7.92 |
| Baseline CVC | 1st | 0.27 $\pm$ 0.20 | 0.26 $\pm$ 0.16 | 0.25 $\pm$ 0.18 | 0.24 $\pm$ 0.13 |
| | 2nd | 0.20 $\pm$ 0.08 | 0.22 $\pm$ 0.08 | 0.21 $\pm$ 0.11 | 0.21 $\pm$ 0.10 |
| Peak CVC | 1st | 2.13 $\pm$ 0.54 | 1.82 $\pm$ 0.48 | 1.89 $\pm$ 0.55 | 1.70 $\pm$ 0.62 |
| | 2nd | 2.10 0.53 | 1.84 $\pm$ 0.46 | 1.85 $\pm$ 0.63 | 1.71 $\pm$ 0.53 |
| Peak CVC change | 1st | 1.87 $\pm$ 0.56 | 1.57 $\pm$ 0.49 | 1.64 $\pm$ 0.55 | 1.46 $\pm$ 0.61 |
| | 2nd | 1.90 0.51 | 1.62 $\pm$ 0.46 | 1.64 $\pm$ 0.62 | 1.50 $\pm$ 0.49 |
| % of peak CVC change | 1st | 972 $\pm$ 535 | 846 $\pm$ 511 | 819 $\pm$ 347 | 799 $\pm$ 519 |
| | 2nd | 1040 $\pm$ 395 | 831 $\pm$ 451 | 888 $\pm$ 342 | 815 $\pm$ 275 |
| 4 min AUC (PU) | 1st | 30707 $\pm$ 7445 | 26335 $\pm$ 7192 | 26203 $\pm$ 8010 | 23597 $\pm$ 7813 |
| | 2nd | 29220 $\pm$ 7172 | 26594 $\pm$ 6426 | 25459 $\pm$ 6272 | 23815 $\pm$ 7643 |
| Time to peak flux (sec) | 1st | 156.4 $\pm$ 33.2 | 163.1 $\pm$ 30.0 | 157.5 $\pm$ 32.2 | 139.8 $\pm$ 36.6 |
| | 2nd | 161.0 $\pm$ 41.0 | 150.6 $\pm$ 34.7 | 150.2 $\pm$ 34.5 | 143.3 $\pm$ 35.7 |

No difference was found in any of the comparisons between the 1st and 2nd test. RF, RE, LF, and LE indicate the right and left flexor and extensor recording sites, respectively.

The baseline CVC data obtained from the 1.5-3.0 minutes average showed no differences from the 3.0-5.0 minutes average; neither is the peak CVC calculated from the 30 seconds average different from the 60 seconds average (data not shown). Therefore, only the data calculated from the 1.5-3.0 minutes baseline average and peak 30 seconds average are presented and were used in the subsequent analysis. The results revealed no differences in the CVC data in any of the recording sites between the first and second tests. However, baseline blood flux and baseline CVC revealed relatively lower values and smaller SDs in the second measurement compared with the first measurement. The time to peak flux ranged from 90 to 209 seconds and averaged 139.8 $\pm$ 36.6 to 163.1 $\pm$ 30.0 seconds.

Intrasession reproducibility was assessed by CV and ICC from the Day 1 data in the 30 minutes acclimation group. The reproducibility of the different forms of data expression, including baseline CVC, peak CVC, peak CVC change, percentage of peak CVC change, 4 minutes AUC, and time to peak flux, were calculated (Table 3).

The data indicated that baseline CVC (CV = 30.09-41.38%, ICC = -0.42-0.70), percentage of peak CVC change (CV = 34.84-42.56%, ICC = 0.44-0.76), and time to peak flux (CV = 17.51-21.38%, ICC = 0.08-0.66) were not reproducible. The reproducibility of peak CVC (CV = 10.94-17.31%, ICC = 0.77-0.91), peak CVC change (CV = 13.75-18.12%, ICC = 0.80-0.92), and 4 minutes AUC (CV = 12.04-18.70%, ICC = 0.60-0.94) was moderate. In conclusion, intrasession reproducibility was acceptable for peak CVC, peak CVC change, and 4 minutes AUC.

Table 3. Intrasession reproducibility of cutaneous local thermal hyperemia using different forms of data expression.

|  | RF | | RE | | LF | | LE | |
|---|---|---|---|---|---|---|---|---|
|  | CV(%) | ICC | CV(%) | ICC | CV(%) | ICC | CV(%) | ICC |
| Baseline CVC | 41.38 | -0.42 | 37.95 | 0.12 | 31.59 | 0.48 | 30.09 | 0.70 |
| Peak CVC | 16.02 | 0.77 | 10.94 | 0.85 | 17.31 | 0.78 | 13.89 | 0.91 |
| Peak CVC change | 14.30 | 0.86 | 13.75 | 0.88 | 18.12 | 0.80 | 15.91 | 0.92 |
| % of peak CVC change | 41.74 | 0.56 | 42.56 | 0.76 | 34.84 | 0.75 | 36.10 | 0.44 |
| 4 min AUC | 18.70 | 0.60 | 12.04 | 0.85 | 18.37 | 0.78 | 12.41 | 0.94 |
| Time to peak flux | 21.38 | 0.31 | 20.00 | 0.08 | 17.51 | 0.43 | 17.90 | 0.66 |

RF, RE, LF, and LE indicate the right and left flexor and extensor recording sites, respectively.

**Example 3: Intersession reproducibility.**

Intersession reproducibility was assessed using CV and ICC from the first measurement on each day in the 30- and 60-min acclimation groups. The data expression was the same as intrasession reproducibility. The data from the 60 minutes acclimation group were more reproducible than those from the 30 minutes acclimation group. Therefore, only the data from the 60 minutes acclimation group are presented in Table 4.

Table 4. Intersession reproducibility of cutaneous local thermal hyperemia using different forms of data expression.

|  | RF | | RE | | LF | | LE | |
|---|---|---|---|---|---|---|---|---|
|  | CV (%) | ICC | CV (%) | ICC | CV (%) | ICC | CV (%) | ICC |
| Baseline CVC | 40.50 | 0.23 | 33.14 | 0.51 | 32.91 | 0.51 | 30.70 | 0.59 |
| Peak CVC | 14.65 | 0.88 | 21.92 | 0.76 | 22.60 | 0.65 | 24.08 | 0.74 |
| Peak CVC change | 24.84 | 0.68 | 24.67 | 0.68 | 24.66 | 0.66 | 24.58 | 0.74 |
| % of peak CVC change | 50.50 | 0.71 | 46.51 | 0.43 | 38.24 | 0.68 | 31.08 | 0.56 |
| 4 min AUC | 14.91 | 0.88 | 20.21 | 0.72 | 24.01 | 0.63 | 24.50 | 0.54 |
| Time to peak flux | 18.80 | 0.56 | 17.48 | 0.51 | 16.71 | 0.29 | 21.14 | 0.24 |

RF, RE, LF, and LE indicate the right and left flexor and extensor recording sites, respectively.

The data showed that baseline CVC (CV = 30.70-40.50%, ICC = 0.23-0.59), percentage of peak CVC change (CV = 31.08-50.50%, ICC = 0.43-0.71), and time to peak flux (CV = 16.71-21.14%, ICC = 0.24-0.56) were not reproducible. Intersession reproducibility was acceptable when expressed as peak CVC (CV = 14.65-24.08%, ICC = 0.65-0.88) and peak CVC change (CV = 24.58-24.84%, ICC = 0.66-0.74), and 4 minutes AUC (CV = 14.91-24.50%, ICC = 0.54-0.88). Intersession reproducibility of peak CVC in the 30 minutes acclimation group was poor (CV = 16.76-24.89%, ICC = -0.11-0.77) and in the 60 minutes acclimation group was moderate (CV = 14.65-24.08%, ICC = 0.65-0.88). The time to peak flux ranged from 90 to 209 seconds and averaged $139.8 \pm 36.6$ to $163.1 \pm 30.0$ s.
Data from Examples 2 and 3 demonstrated that intrasession and intersession reproducibility could be acceptable when the recording sites were located by predefined rules using anatomical landmarks and the data were expressed as peak CVC, peak CVC change, and 4 minutes AUC.

**Example 4: Effect of acclimation on reproducibility**

To explore the effect of the acclimation period on the reproducibility of short-term heating cutaneous local thermal hyperemia, both intrasession and intersession reproducibility was analyzed and is presented in Table 5. Intrasession reproducibility is expressed as CV and ICC from the data on Day 1, and only the reproducible forms of data expression (i.e., peak CVC, peak CVC change (ΔCVC), and 4 minutes AUC) are presented.

Table 5. Intrasession and intersession reproducibility of cutaneous local thermal hyperemia in 30- and 60-min acclimation groups.

| Reproducibility | Data | Acclimation | RF | | RE | | LF | | LE | |
|---|---|---|---|---|---|---|---|---|---|---|
| | | | CV (%) | ICC | CV (%) | ICC | CV (%) | ICC | CV (%) | ICC |
| Intrasession | Peak | 30 min | 16.02 | 0.77 | 10.94 | 0.85 | 17.31 | 0.78 | 13.89 | 0.91 |
| | CVC | 60 min | 15.32 | 0.92 | 18.33 | 0.79 | 11.41 | 0.94 | 19.32 | 0.90 |
| | ΔCVC | 30 min | 14.30 | 0.86 | 13.75 | 0.88 | 18.12 | 0.80 | 15.91 | 0.92 |
| | | 60 min | 19.28 | 0.91 | 23.69 | 0.67 | 11.23 | 0.94 | 22.31 | 0.90 |
| | 4 min | 30 min | 18.70 | 0.60 | 12.04 | 0.85 | 18.37 | 0.78 | 12.41 | 0.94 |
| | AUC | 60 min | 15.44 | 0.88 | 14.76 | 0.86 | 14.34 | 0.89 | 18.69 | 0.91 |
| Intersession | Peak | 30 min | 16.76 | 0.77 | 24.89 | -0.11 | 21.97 | -0.04 | 21.61 | 0.53 |
| | CVC | 60 min | 14.65 | 0.88 | 21.92 | 0.76 | 22.60 | 0.65 | 24.08 | 0.74 |
| | ΔCVC | 30 min | 19.31 | 0.66 | 19.75 | 0.34 | 21.22 | 0.20 | 25.93 | 0.32 |
| | | 60 min | 24.84 | 0.68 | 24.67 | 0.68 | 24.66 | 0.66 | 24.58 | 0.74 |
| | 4 min | 30 min | 19.70 | 0.60 | 22.24 | 0.35 | 25.38 | 0.11 | 26.43 | 0.34 |
| | AUC | 60 min | 14.91 | 0.88 | 20.21 | 0.72 | 24.01 | 0.63 | 24.50 | 0.54 |

RF, RE, LF, and LE indicate the right and left flexor and extensor recording sites, respectively.

Intersession reproducibility is expressed as CV and ICC from the data from the first measurement on Day 1 and Day 2, and only the reproducible form of data expression (i.e., peak CVC) is presented. The data indicated that intersession reproducibility was better in the 60 minutes acclimation group compared with the 30 minutes acclimation group using peak CVC, and only the data from the 60 minutes acclimation group was reproducible. The data showed equally acceptable intrasession reproducibility between the 30- and 60-minute acclimation groups in all forms of data expression.

These results demonstrated that intersession reproducibility was better with a 60-min acclimation period than a 30-min acclimation period, and only the 60-min acclimation period yielded reproducible data. By contrast, intrasession reproducibility was equally acceptable in both the 30- and 60-min acclimation groups. Therefore, the intrasession test could be performed after a 30 minutes acclimation period, which is relatively time-saving; however, the intersession test should be conducted after a longer acclimation period, such as 60 minutes.

## Example 5: Comparing reproducibility between males and females

Since no differences were found in the intrasession reproducibility of short-term heating cutaneous local thermal hyperemia between the 30- and 60-minute acclimation groups, data from the two groups were pooled together for the comparison of intrasession reproducibility between males and females (n = 20 each). Only the reproducible forms of data expression (i.e., peak CVC, peak CVC change (ΔCVC), and 4 minutes AUC) were compared. The results, as summarized in Table 6, indicated equally acceptable intrasession reproducibility between males and females in all forms of data expression.

The intersession reproducibility of short-term heating cutaneous local thermal hyperemia was acceptable in the 60-min acclimation group but not in the 30-min acclimation group. Therefore, we compared intersession reproducibility between males and females only in the 60-min acclimation group (n = 10 each). Only the reproducible form of data expression (i.e., peak CVC, peak CVC change and 4 minutes AUC) was compared. The results indicated that intersession reproducibility was equally acceptable for both males and females (Table 6).

Although female reproductive hormones have been reported to influence the local thermal control of skin blood flow, the present example indicated that both intrasession and intersession reproducibilities were equally acceptable in males and females. Therefore, this protocol may be applied to studies of both genders.

Table 6. Comparison of intrasession and intersession reproducibility between males and females.

| Reproducibility | Data | Gender | RF | | RE | | LF | | LE | |
|---|---|---|---|---|---|---|---|---|---|---|
| | | | CV (%) | ICC | CV (%) | ICC | CV (%) | ICC | CV (%) | ICC |
| Intrasession | Peak | Male | 16.33 | 0.68 | 13.18 | 0.81 | 14.42 | 0.90 | 19.87 | 0.85 |
| | CVC | Female | 14.98 | 0.91 | 16.79 | 0.81 | 14.89 | 0.84 | 13.10 | 0.92 |
| | ΔCVC | Male | 15.13 | 0.79 | 14.81 | 0.80 | 14.03 | 0.92 | 22.54 | 0.90 |

(continued)

| Reproducibility | Data | Gender | RF | | RE | | LF | | LE | |
|---|---|---|---|---|---|---|---|---|---|---|
| | | | CV (%) | ICC | CV (%) | ICC | CV (%) | ICC | CV (%) | ICC |
| Intersession | | Female | 18.63 | 0.90 | 23.04 | 0.74 | 16.05 | 0.84 | 15.58 | 0.91 |
| | 4 min | Male | 18.44 | 0.61 | 12.88 | 0.85 | 14.98 | 0.90 | 19.76 | 0.89 |
| | AUC | Female | 15.76 | 0.83 | 14.04 | 0.86 | 17.85 | 0.63 | 10.62 | 0.94 |
| | Peak | Male | 16.59 | 0.82 | 24.23 | 0.72 | 21.13 | 0.75 | 21.74 | 0.72 |
| | CVC | Female | 14.84 | 0.93 | 22.65 | 0.84 | 23.39 | 0.58 | 23.97 | 0.74 |
| | △CVC | Male | 16.13 | 0.72 | 22.81 | 0.80 | 17.06 | 0.89 | 24.64 | 0.82 |
| | | Female | 18.93 | 0.88 | 24.07 | 0.74 | 19.15 | 0.82 | 19.68 | 0.87 |
| | 4 min | Male | 19.24 | 0.71 | 19.98 | 0.85 | 18.68 | 0.88 | 21.86 | 0.83 |
| | AUC | Female | 16.86 | 0.81 | 19.84 | 0.86 | 18.95 | 0.70 | 18.62 | 0.91 |

RF, RE, LF, and LE indicate the right and left flexor and extensor recording sites, respectively.

**Example 6: Conclusive indices of intrasession or intersession reproducibility expressed by peak CVC**

Since peak CVC was the common reproducible index for both intrasession and intersession assessments, Table 7 provides all of the indices of peak CVC. The mean differences in peak CVC ranges from -0.04 to 0.02 and from -0.05 to 0.08 for the intrasession and intersession tests-retests, respectively. The MDCs ranges from 0.44 to 0.76 and from 0.61 to 0.93, respectively. The MDCs from most recording sites were higher in the intersession assessment than those in the intrasession assessment.

Table 7. Conclusive indices of intrasession or intersession reproducibility expressed as peak CVC.

| Reproducibility | Recording site | First test | Second test | Difference | CV(%) | ICC | SEM | MDC |
|---|---|---|---|---|---|---|---|---|
| Intrasession | RF | 2.1310.54 | 2.10 ± 0.53 | -0.03 ± 0.48 | 16.02 | 0.77 | 0.25 | 0.70 |
| | RE | 1.82 ± 0.48 | 1.84 ± 0.46 | 0.02 ± 0.33 | 10.94 | 0.85 | 0.18 | 0.50 |
| | LF | 1.89 ± 0.55 | 1.85 ± 0.63 | -0.04 ± 0.50 | 17.31 | 0.78 | 0.27 | 0.76 |
| | LE | 1.70 ± 0.62 | 1.71 ± 0.53 | 0.01 ± 0.31 | 13.89 | 0.91 | 0.16 | 0.44 |
| Intersession | RF | 1.94 ± 0.67 | 1.97 ± 0.61 | 0.03 ± 0.40 | 14.65 | 0.88 | 0.22 | 0.61 |
| | RE | 1.78 ± 0.71 | 1.86 ± 0.61 | 0.08 ± 0.58 | 21.93 | 0.76 | 0.32 | 0.89 |
| | LF | 1.73 ± 0.60 | 1.68 ± 0.54 | -0.05 ± 0.58 | 22.60 | 0.65 | 0.33 | 0.93 |
| | LE | 1.73 ± 0.66 | 1.73 ± 0.60 | 0.00 ± 0.57 | 24.08 | 0.74 | 0.32 | 0.89 |

RF, RE, LF, and LE indicate the right and left flexor and extensor recording sites, respectively. CV, coefficient of variation (%); ICC, intra-class correlation coefficient; SEM, standard error of measurement; MDC, minimal detectable change.

In sum, working examples 1 to 6 confirmed that the intrasession and intersession reproducibility of short-term heating cutaneous local thermal hyperemia with 5 minutes heating using single-point LDF reached an acceptable level if the recording sites were fixed and the data were expressed as peak CVC, peak CVC change, and 4 min AUC. Minimizing the recording time to obtain acceptable reproducibility for cutaneous local thermal hyperemia testing is a major feature of the present disclosure. This relatively simple method for cutaneous local thermal hyperemia testing may significantly improve accessibility; and thereby widely increase the application of cutaneous local thermal hyperemia. The advantages of the method of this invention manifested in the following aspects. First, when the data are expressed as peak CVC, peak CVC change, and 4 min AUC without the involvement of plateau CVC, the heating period could be shortened to 5 min. Second, peak CVC may be represented by the average CVC of 30 seconds around the peak flux, which is nearly the same as that of 60-second average. Additionally, all of the values of the time to peak flux are under 210 seconds. Therefore, the baseline measurement for 2-5 minutes and heating for 4 minutes are sufficient to obtain enough data for further analysis. Using this protocol, the heating temperature is maintained at 42°C for only 2.5 minutes (from 90 to 240 seconds during the heating interval), and a shorter hyperemia reaction will be induced, hence blood vessels may recover more quickly. This allows for a further reduction in the time interval between two tests in a single session. A shorter test-retest interval not only saves time but also provides flexibility in the selection of the time interval. It also benefits the assessment of axon reflex-related microvascular function, which is considered to be affected by sensory and sympathetic functions. The total time period required for recording one site is as short as 6 minutes, and this time-saving method enables testers such as operators to record multiple sites sequentially without utilizing more equipments or other complicated equipment.

Additionally, placing the probes in the same position precisely is difficult in repeated tests. The results with acceptable intersession reproducibility also suggest that the variability of capillary density makes little contribution to the initial maximum of cutaneous local thermal hyperemia in a limited area. The reason for choosing the recording sites on the radial side of both the flexor and extensor forearms is that these sites are relatively easily accessible, and the participants may feel more comfortable by placing their hands on their abdomen in a supine position.

In conclusion, fixing the recording site improved the intrasession and intersession reproducibility of short-term heating cutaneous local thermal hyperemia on both the flexor and extensor aspects of the forearm using single-point LDF and may increase usability by shortening the heating period from more than 30 minutes to 4 minutes. A 30-min acclimation period is appropriate for intrasession assessment, but a longer acclimation period (e.g., 60 min) may be needed for intersession assessment. This new protocol can be applied to both males and females.

**Example 7: Normative value of different vasodilation parameters at various sampling sites**

To determine the normative values of different vasodilation parameters at various sampling site, another cohort of healthy male and female volunteers (n = 12 each), aged 20-40 years, were recruited from website advertisements. The exclusion criteria and restrictions for the participants were the same as those recited in Example 1, above.

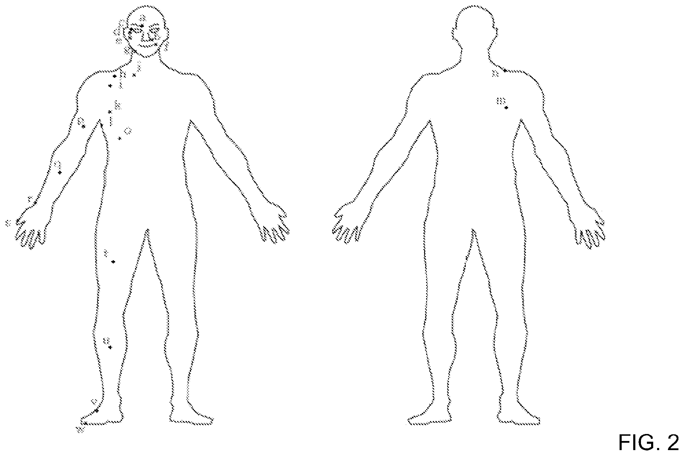

The sampling sites used in the present example are illustrated in Figure 2, and the position of each sampling site is as follows. Point (a) locates at the right or left inner eyebrow. Point (b) indicates the para-nasal site which is about 1-cm lateral to the left or right nasal alar. Point (c) is the para-canthus site which is about 1-cm lateral to the external canthus formed by the meeting of the upper and lower eyelids at either side of the eye. Point (d) is the preauricular site which is about 1-cm anterior to the left or right ear canal. Point (e) is the pre-ear lobe site about 1.5-cm below the point (d). Point (f) is located at about 1 cm lateral to either corners of the mouth. Point (g), the jaw, locates at the meeting point of the vertical line from the middle of the pupil (right or left) and the mandible. The supraclavicular point (h) is slightly above the medial 1/3 of the clavicle, while the infraclavicular point (i) locates at upper aspect of upper 1/2 of the clavicular fiber of pectoralis major muscle. The parasternal point (j) is located at the intersection of sternum and clavicle. Point (k), the lateral chest site, is situated at the upper 1/3 of the sternal fiber of pectoralis major muscle. The axilla point (l) is at about 4-cm below the axillary fossa. Point (m) is the suprascapular site which locates at the 1/3 of the infraspinatous from the middle boarder of scapula. Point (n) locates at the shoulder which is 1/2 of the upper aspect of the upper trapezius. The sub-breast point (o) is located at about 3-cm below the left or right nipple. Point (p) and point (q) are respectively situated at 1/2 of the biceps brachii (mid-arm) and 1/2 of the flexor carpi radialis (mid-forearm). Point (r) on the wrist locates at the radial part of the flexor retinaculum of wrist. Point (s) on the thumb locates at 1/2 of the lateral aspect of distal phalange of thumb. Point (t) and point (u) are respectively situated at 1/2 of the rectus femoris (mid-tight) and 1/2 of the tibialis anterior (mid-leg). Point (v) on the dorsal foot locates at the joint between the lateral cuneiform bone and metatarsal bone of great toe. Point (w) on the big toe locates at 1/2 of the middle aspect of distal phalange of great toe.

Upon arrival in the laboratory, the subjects were arranged on chairs in a temperature- and humidity-controlled laboratory (40-60% humidity, $25 \pm 1°C$) for acclimation of 30 minutes. After the acclimation period, the thermal hyperemia test was performed at specified sampling sites. The participants were supine during the entire test period. Cutaneous blood flow was measured by LDF (Moor DRT4, Moor Instruments Ltd, Axminster, Devon, UK). For each measurement, blood pressure was recorded continuously with a non-invasive blood pressure monitor (CareTaker, Empirical Technologies Corporation, Charlottesville, Virginia, USA) on the middle finger of the dominant hand throughout the procedures. The sampling and data processing procedures taught in Example 1, above, also applied in the present example. Results are summarized in Table 8.

Table 8. Normative value of $CVC_{I, max}$, $\Delta CVC$ and initial AUC at various sampling sites.

| Sampling Site | $CVC_{I, max}$ | $\Delta CVC$ | Initial AUC |
|---|---|---|---|
| a. inner eyebrow | $2.26 \pm 0.92$ | $1.92 \pm 0.61$ | $36514 \pm 8721$ |
| b. para-nasal | $2.76 \pm 0.50$ | $2.39 \pm 0.45$ | $40126 \pm 9093$ |
| c. para-canthus | $3.29 \pm 0.21$ | $2.51 \pm 0.50$ | $45982 \pm 9128$ |
| d. preauricular | $2.26 \pm 0.57$ | $1.89 \pm 0.78$ | $33549 \pm 7954$ |
| e. pre-ear lobe | $2.22 \pm 0.65$ | $1.86 \pm 0.69$ | $31202 \pm 8076$ |
| f. mouth corner | $2.21 \pm 0.35$ | $1.95 \pm 0.34$ | $30078 \pm 7892$ |
| g. jaw | $1.67 \pm 0.26$ | $1.31 \pm 0.35$ | $20000 \pm 5781$ |

(continued)

| Sampling Site | $CVC_{I, max}$ | $\Delta CVC$ | Initial AUC |
|---|---|---|---|
| h. supraclavicular | 1.75 ± 0.63 | 1.47 ± 0.58 | 26626 ± 6926 |
| i. infraclavicular | 1.28 ± 0.35 | 1.09 ± 0.33 | 18326 ± 4851 |
| j. parasternal | 1.75 ±0.63 | 1.55 ± 0.62 | 26353 ± 6488 |
| k. lat. chest | 1.33 ± 0.46 | 1.13 ± 0.33 | 19863 ± 4955 |
| l. axilla | 1.33 ± 0.26 | 1.15 ± 0.27 | 19918 ± 4137 |
| m. suprascapular | 1.13 ± 0.28 | 1.00 ± 0.21 | 17670 ± 4371 |
| n. shoulder | 1.20 ± 0.27 | 1.01 ± 0.22 | 18370 ± 4143 |
| o. sub-breast | 0.90 ± 0.30 | 0.69 ± 0.22 | 13115 ± 3397 |
| p. mid-arm | 1.21 ± 0.26 | 0.98 ± 0.20 | 17265 ± 4001 |
| q. mid-forearm | 1.35 ± 0.28 | 1.17 ± 0.29 | 25849 ± 5911 |
| r. wrist | 1.32 ± 0.29 | 1.12 ± 0.29 | 20862 ± 4839 |
| s. thumb | 2.08 ± 0.36 | 1.74 ± 0.41 | 30525 ± 6971 |
| t. mid-thigh | 1.06 ± 0.26 | 0.88 ± 0.19 | 15775 ± 4102 |
| u. mid-leg | 1.11 ± 0.21 | 0.97 ± 0.22 | 19345 ± 5137 |
| v. dorsal foot | 0.97 ± 0.24 | 0.69 ± 0.14 | 15046 ± 3423 |
| w. big toe | 0.99 ± 0.31 | 0.72 ± 0.20 | 15912 ± 4428 |

**Example 8: Clinical analysis of cutaneous local thermal hyperemia response**

As described hereinabove, microvascular dysfunction may be associated with wide variety of diseases/disorders. Therefore, the initial maximum CVC, maximum CVC change, and 4 minutes AUC data obtained in accordance with embodiments of the present disclosure are useful tools for the diagnosis of these diseases, documentation of the disease progression, effectiveness of a potential or prescribed therapy, and unanticipated/undesirable effects of a given therapy. Below are several clinical examples from healthy subjects or subjects suffering from various sensory or sympathetic disorders. Vascular-related measurements and values of vasodilation parameters were measured and obtained according to the procedures set forth in Example 7, above.

8.1 Bilateral analysis of anatomically symmetric points of healthy subject

Male healthy subject (22 y/o; 73 kg) was sampled at the right and left preauricular position (point (d) in Figure 2), and the results are summarized in Table 9.

Table 9 $CVC_{I, max}$, $\Delta CVC$ and initial AUC at anatomically symmetric sampling sites of healthy subject.

| Sampling Site | $CVC_{I, max}$ | $\Delta CVC$ | Initial AUC |
|---|---|---|---|
| Preauricular, right | 2.62 | 2.25 | 36001 |
| Preauricular, left | 2.79 | 2.34 | 39872 |

The normative values of $CVC_{I, max}$, $\Delta CVC$ and Initial AUC at the preauricular position are 2.26 ± 0.57, 1.86 ± 0.69 and 33549 ± 7954 (see, Table 8), respectively, and the values of these parameter obtained from the subject fall within the normative range. These data reveal that the subject's sensory and/or sympathetic function at the sampled site is normal, indicating that the subject may be a healthy subject with no observable vascular and/or neurological dysfunctions.

Theoretically, the sensory and/or sympathetic functions between the anatomically symmetric positions are substantially the same. The data in Table 9 demonstrate that there are no statistically significant differences between the values obtained from both sides. This result also echoes the result in Example 2, above, which illustrates that the present method exhibits satisfactory intrasession reproducibility.

8.2 Bilateral analysis of anatomically symmetric points of subject having left trigeminal neuralgia

Female subject (53 y/o; 67 kg) suffering from trigeminal neuralgia at the left side was sampled at the right and left pre-ear lobes (point (e) in Figure 2), and the results are summarized in Table 10.

Table 10 $CVC_{I, max}$, $\Delta CVC$ and initial AUC at anatomically symmetric sampling sites of subject having left trigeminal neuralgia.

| Sampling Site | $CVC_{I, max}$ | $\Delta CVC$ | Initial AUC |
|---|---|---|---|
| Pre-ear lobe, right | 2.19 | 1.96 | 31304 |
| Pre-ear lobe, left | 4.28 | 3.89 | 61831 |

Trigeminal neuralgia causes hyperalgia in which the sensory nerve is sensitized and the cutaneous local thermal hyperemia response is enhanced. In this case, the values of three vasodilation parameters (4.28, 3.89 and 61831, respectively) obtained from the left side of the subject were almost doubled from the mean normative values (2.22, 1.86, and 31202, respectively; see, Table 8), whereas the values obtained from the right side (2.19, 1.96 and 31304, respectively) were within the normative ranges. These data indicate that the cutaneous local thermal hyperemia response at the subject's left side was greatly enhanced; suggesting that the sensory nerve associated with the subject's left face might be sensitized. This result coincides with the fact that the subject suffers from left trigeminal neuralgia.

8.3 Bilateral analysis of anatomically symmetric points of subject having right facial palsy

Female subject (43 y/o; 52 kg) suffering from facial palsy at the right side was sampled at the right and left para-nasal sites (point (b) in Figure 2), and the results are summarized in Table 11.

Table 11 $CVC_{I, max}$, $\Delta CVC$ and initial AUC at anatomically symmetric sampling sites of subject having right facial palsy.

| Sampling Site | $CVC_{I, max}$ | $\Delta CVC$ | Initial AUC |
|---|---|---|---|
| Para-nasal, right | 2.12 | 1.77 | 30525 |
| Para-nasal, left | 2.77 | 2.26 | 38389 |

Facial palsy is the paralysis of part of the face caused by non-functioning of the nerve that controls the muscles of the face, especially the muscles around the eye and to the mouth. The data in Table 11 indicate that the values of three vasodilation parameters (2.12, 1.77 and 30525, respectively) obtained from the right side of the subject were slightly lower than the normative ranges (2.76 $\pm$ 0.50, 2.39 $\pm$ 0.45, and 40126 $\pm$ 9093, respectively; see, Table 8), whereas the values obtained from the left side (2.77, 2.26 and 38389, respectively) were within the normative ranges. Accordingly, it is inferred that the subject's right facial nerve may be damaged. This result is in line with the clinical diagnosis of the subject.

8.4 Bilateral analysis of anatomically symmetric points of subject having post-herpetic neuralgia

Male subject (75 y/o; 82 kg) suffering from post-herpetic neuralgia (PHN) at the left face was sampled at the right and left para-nasal sites (point (b) in Figure 2), and the results are summarized in Table 12.

Table 12 $CVC_{I, max}$, $\Delta CVC$ and initial AUC at anatomically symmetric sampling sites of PHN subject.

| Sampling Site | $CVC_{I, max}$ | $\Delta CVC$ | Initial AUC |
|---|---|---|---|
| Para-nasal, right | 2.46 | 2.06 | 38041 |
| Para-nasal, left | 1.42 | 1.22 | 22097 |

Post-herpetic neuralgia is a nerve pain due to damage caused by the varicella zoster virus. The data in Table 12 indicate that the values of three vasodilation parameters (1.42, 1.22 and 22097, respectively) obtained from the subject's left face were lower than the normative ranges (2.76 $\pm$ 0.50, 2.39 $\pm$ 0.45, and 40126 $\pm$ 9093, respectively; see, Table 8), whereas the values obtained from the right face (2.46, 2.06 and 38041, respectively) were within the normative ranges. These data reveal that the left facial nerve may be damaged, which is in agreement with the diagnosis of left facial PHN.

8.5 Bilateral analysis of anatomically symmetric points of subject having myofascial pain syndrome

Male subject (38 y/o; 69 kg) suffering from myofascial pain syndrome (MPS) at the right shoulder was sampled at the right and left shoulder (point (n) in Figure 2), and the results are summarized in Table 13.

Table 13 $CVC_{I, max}$, $\Delta CVC$ and initial AUC at anatomically symmetric sampling sites of MPS subject.

| Sampling Site | $CVC_{I, max}$ | $\Delta CVC$ | Initial AUC |
|---|---|---|---|
| Shoulder, right | 0.79 | 0.66 | 12530 |
| Shoulder, left | 1.32 | 1.08 | 19469 |

Myofascial pain syndrome is characterized by chronic pain caused by multiple trigger points and fascial constrictions. A myofascial trigger point is a self-sustaining irritable area in a taut band of muscle fiber that causes gradual shortening of the muscle interfering with normal muscle function, and causing pressure on the surrounding nerves. In addition, the blood circulation is impaired in the shortened and aching muscle. Hence, the cutaneous local thermal hyperemia response around the myofascial trigger point may be jeopardized. The data in Table 13 indicate that the values of three vasodilation parameters (0.79, 0.66 and 12530, respectively) obtained from the subject's right shoulder were lower than the normative ranges ($1.20 \pm 0.27$, $1.01 \pm 0.22$, and $18370 \pm 4143$, respectively; see, Table 8), whereas the values obtained from the left shoulder (1.32, 1.08 and 19469, respectively) were within the normative ranges. These data indicate that the nervous function and blood circulation associated with the subject's left shoulder nerve may be abnormal, confirming the diagnosis of left shoulder MPS.

8.6 Bilateral analysis of anatomically symmetric points at various sampling sites of subject having diabetes

Female subject (82 y/o; 57 kg) suffering from diabetes was sampled at the mid-leg, dorsal foot and big toe (points (u), (v) and (w) in Figure 2) on both the right and left sides, and the results are summarized in Table 14.

Table 14 $CVC_{I, max}$, $\Delta CVC$ and initial AUC at anatomically symmetric sampling sites of diabetic subject.

| Sampling Site | $CVC_{I, max}$ | $\Delta CVC$ | Initial AUC |
|---|---|---|---|
| Mid-forearm, right | 1.31 | 1.08 | 23042 |
| Mid-forearm, left | 1.33 | 1.10 | 24574 |
| Wrist, right | 1.29 | 1.13 | 19887 |
| Wrist, left | 1.30 | 1.09 | 20128 |
| Thumb, right | 1.02 | 0.82 | 16852 |
| Thumb, left | 1.05 | 0.85 | 16325 |
| Mid-leg, right | 1.07 | 0.93 | 18962 |
| Mid-leg, left | 1.14 | 1.02 | 22476 |
| Dorsal foot, right | 0.71 | 0.48 | 10548 |
| *Dorsal foot, left | 0.68 | 0.51 | 11636 |
| Big toe, right | 0.39 | 0.27 | 7481 |
| Big toe, left | 0.44 | 0.28 | 7970 |

Diabetes is a metabolic disease in which patients have high blood glucose level. Complications of diabetes include peripheral neuropathy and decreased blood flow to the legs and feet, leading to amputation if not treated. The impairment of sensory nerves resulted from peripheral neuropathy often starts from the distal limbs and gradually extends to more proximal part when the disease progresses. In the present case, the most distal sampling site of upper extremity was the right and left thumb, and the vasodilation-related values obtained from the thumbs of the subject were only about half of the mean normative values at the same sampling site (2.08, 1.74 and 30525, respectively; see, Table 8). Moving from the thumb to the wrist, it was observed that the sampled values were still lower than the normative ranges ($1.32 \pm 0.29$, $1.12 \pm 0.29$, $20862 \pm 4839$, respectively; see, Table 8), yet they were close to the lower limits of the normative ranges. Proceeding to a more proximal part, the mid-forearm, the sampled values were within the normative ranges and quite close to the mean normative values (1.35, 1.17, 25849, respectively; see, Table 8). In addition, the most distal sampling site of lower extremity was the right and left big toes, and the vasodilation-related values obtained from the big toes of the subject were less than half of the mean normative values at the same sampling site (0.99, 0.72 and 15912, respectively; see, Table 8). Moving from the big toe to the dorsal foot, it was observed that the sampled values were still lower than the normative ranges (0.97 ± 0.24, 0.69 ± 0.14 and 15046 ± 3423, respectively; see, Table 8), yet they were close to the lower limits of the normative ranges. Proceeding to a more proximal part, the mid-leg, the sampled values were within the normative ranges and quite close to the mean normative values (1.11, 0.97 and 19345, respectively; see, Table 8). The comparative value between the distal and proximal sampling sites were presented in Table x and compared with normative value. The relative values of the most distal sampling site to the other proximal sites, i.e. thumb/mid-forearm, thumb/wrist, big toe/mid-leg, and big toe/dorsal foot were lower than normative values. These results indicate that the cutaneous local thermal hyperemia response at the distal limb is weaker than that at the proximal limb. Therefore, the present method is suitable for use in the evaluation of the progression of the disease, as well as the efficacy of the treatment.

8.7 Bilateral analysis of anatomically symmetric points at various sampling sites before and after chemical intervention

Female subject (26 y/o; 45 kg) suffering from panic disorder was sampled at the infraclavicular, parasternal and axilla sites (points (i), (j) and (l) in Figure 2) on both the right and left sides before and after the administration of β-blocker. Specifically, the post-β-blocker sampling procedure was performed at 30 minutes after the oral administration of β-blocker, propranolol, (10 mg). Results are summarized in Table 15.

Table 15 $CVC_{I, max}$, ΔCVC and initial AUC at anatomically symmetric sampling sites before and after chemical intervention.

| Sampling Site | $CVC_{I, max}$ | | ΔCVC | | Initial AUC | |
|---|---|---|---|---|---|---|
| | Before | After | Before | After | Before | After |
| Infraclavicular, right | 2.63 | 1.95 | 2.42 | 1.76 | 44963 | 33797 |
| Infraclavicular, left | 1.44 | 1.03 | 1.25 | 0.88 | 21336 | 17298 |
| Parasternal, right | 3.21 | 2.53 | 2.98 | 2.36 | 56902 | 47602 |
| Parasternal, left | 2.04 | 1.45 | 1.87 | 1.28 | 29084 | 26800 |
| Axilla, right | 2.55 | 1.91 | 2.38 | 1.63 | 37460 | 31740 |
| Axilla, left | 1.37 | 1.09 | 1.15 | 0.92 | 21061 | 17019 |

Panic disorder is an anxiety disorder and is characterized by unexpected and repeated episodes of panic attack accompanied by physical symptoms such as racing heart, sweating, breathing problems, weakness or dizziness, feeling hot or a cold chill, tingly or numb hands, chest pain, or stomach pain. Panic disorder is related to autonomic dysfunction. β-blockers that are effective in blocking the peripheral symptoms of anxiety sometimes are prescribed for panic disorders. Theoretically, if a treatment/intervention could significantly alter the function of the sensory or sympathetic nerves, the cutaneous local thermal hyperemia response would also be altered after such treatment/intervention takes effect.

The data in Table 15 indicates that the cutaneous local thermal hyperemia responses at the right and left sides are different from each other. This observation is in accordance with the fact that the peripheral autonomic function is asymmetrical. Specifically, before the chemical intervention, the $CVC_{I, max}$ sampled at the right infraclavicular, right parasternal and right axilla were respectively 2.42, 2.98 and 2.38, which were all substantially higher than the normative ranges (1.28 ± 0.35, 1.75 ± 0.63 and 1.33 ± 0.26, respectively; see, Table 8). In contrast, the $CVC_{I, max}$ sampled at the anatomically symmetric points on the left side were respectively 1.44, 2.04, and 1.37, which were not aberrant from the normative ranges. Regarding the other two vasodilation parameters (ΔCVC and initial AUC), the values obtained from the right-side measurements were also higher than the normative ranges, while the values obtained from the left-side measurements were within the normative ranges.

After the chemical intervention, the hyped response observed in the pre-intervention sampling was alleviated but not completely counteracted. For example, after the chemical intervention, the $CVC_{I, max}$ sampled at the right infraclavicular, right parasternal and right axilla were respectively 1.95, 2.53 and 1.91. Although these values were reduced by approximately 20% to 30% of the pre-intervention values, they were still slightly higher than the upper limits of the normative ranges. On the other hand, regarding the left side of the subject, the $CVC_{I, max}$ values were also reduced from the pre-intervention values, but they were still in the normative ranges.

Unless defined otherwise, all technical and scientific terms used herein have the same meaning as commonly understood by one of the ordinary skill in the art to which this invention belongs. The singular forms "a", "and", and "the" are used herein to include plural referents unless the context clearly dictates otherwise.

The term "treating" as used herein refers to application or administration of the physical and/or chemical intervention to a subject, who has a medical condition, a symptom of the condition, a disease or disorder secondary to the condition, or a predisposition toward the condition, with the purpose to partially or completely alleviate, ameliorate, relieve, delay onset of, inhibit progression of, reduce severity of, and/or reduce incidence of one or more symptoms or features of a particular disease, disorder, and/or condition. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art.

It will be understood that the above description of embodiments is given by way of example only and that various modifications may be made by those It will be understood that the above description of embodiments is given by way of example only and that various modifications may be made by those with ordinary skill in the art. The present invention is as set out in the appended claims.

## Claims

1. A method for obtaining a value of at least one vasodilation parameter representing the cutaneous local thermal hyperemia response of a subject using a laser Doppler-based apparatus (100), comprising:

    (a) performing a baseline measurement at a first sampling site of the subject at a first temperature and obtaining a first baseline red blood cell flux ($RBCF_{BL}$) of the sampling site and a first baseline mean arterial pressure ($P_{BL}$) of the subject;
    (b) raising the temperature of the first sampling site from the first temperature to a second temperature;
    (c) maintaining the second temperature for an initial heating period of 2 to 14 minutes, and recording a plurality of first initial RBCFs ($RBCF_{I, 1-n}$) of the first sampling site at a plurality of time points ($T_{1-n}$) to identify a first initial maximum RBCF ($RBCF_{I, max}$) and recording a first initial mean arterial pressure ($P_I$) of the subject during the initial heating period; and
    (d) calculating the value of the vasodilation parameter, wherein the vasodilation parameter is or is derived from a first initial maximum cutaneous vascular conductance ($CVC_{I, max}$), calculated by the formula of: $CVC_{I, max} = RBCF_{I, max} / P_I$, and the vasodilation parameter derived from the first $CVC_{I, max}$ is a first initial maximum CVC change ($\Delta CVC$) or an initial area-under-the-curve (initial AUC), wherein the $\Delta CVC$ is calculated by the formula of: $\Delta CVC = CVC_{I, max} - (RBCF_{BL} / P_{BL})$; and for the initial AUC, the $RBCF_{I, 1-n}$ are plotted against $T_{1-n}$ to obtain

    a curve having a curve function of $F(X)$, and the initial AUC is calculated by the formula of: $AUC = \int_0^t F(X)dX$,

    where t is equal to or greater than the time at which the $RBCF_{I, max}$ is measured.

2. The method of claim 1, further comprising:

    obtaining a value of a second initial maximum CVC at a second sampling site of the subject at a time interval of 0.5 to 2 hours by repeating the steps (a) to (d).

3. The method of claim 2, wherein the second sampling site is same as the first sampling site.

4. The method of claim 2, wherein the second sampling site is different from the first sampling site.

5. The method of claim 4, wherein the first sampling site and the second sampling site are anatomically symmetric points.

6. The method of claim 1, wherein the baseline measurement is performed for about 2 to 5 minutes.

7. The method of claim 1, wherein the first baseline RBCF, the first initial RBCFs, and the first initial maximum RBCF are measured with a laser Doppler-based probe having a diameter of less than 5 mm.

8. The method of claim 1, wherein the method does not require any subsequent heating step after the initial heating period,

9. The method of any of claims 1, wherein
in the step (b), the temperature is raised at a heating rate of 0.02-0.2°C/sec;
the first temperature is about 28-35°C; and
the second temperature is about 38-44°C.

10. A laser Doppler-based apparatus (100) for obtaining a value of at least one vasodilation parameter representing the cutaneous local thermal hyperemia response of a subject, comprising:

(a) a user interface (110), configured to receive an input from a user and provide an output to the user;
(b) a laser Doppler detector (120), configured to measure at least one vascular-related measurement at a sampling site of the subject during a first sampling procedure, wherein the vascular-related measurement is any of: a first baseline red blood cell flux ($RBCF_{BL}$), a first baseline mean arterial pressure ($P_{BL}$) obtained during a baseline measurement, a first initial RBCF ($RBCF_{I, 1-n}$), a first initial maximum RBCF ($RBCF_{I, max}$), and a first initial mean arterial pressure ($P_I$) obtained during an initial heating period of 2 to 14 minutes;
(c) a heating unit (130), configured to heat the sampling site;
(d) a controller (140), configured to control the laser Doppler detector (120) and the heating unit based on the user input or a default setting;
(e) a computing unit (150), configured to calculate the value of the vasodilation parameter from the at least one vascular-related measurement, wherein the vasodilation parameter is or is derived from a first initial maximum cutaneous vascular conductance ($CVC_{I, max}$), calculated by the formula of: $CVC_{I, max} = RBCF_{I, max} / P_I$, and the vasodilation parameter derived from the first $CVC_{I, max}$ is a first initial maximum CVC change ($\Delta CVC$) or an initial area-under-the-curve (initial AUC), wherein the $\Delta CVC$ is calculated by the formula of: $\Delta CVC = CVC_{I, max} - (RBCF_{BL} / P_{BL})$; and for the initial AUC, the $RBCF_{I, 1-n}$ are plotted against $T_{1-n}$ to obtain a curve having a curve function of F(X), and the initial AUC is calculated by the formula of: $AUC = \int_0^t F(X)dX$, where t is equal to or greater than the time at which the $RBCF_{I, max}$ is measured;
(f) a database (160), comprising a normative value of the vasodilation parameter obtained from a population of healthy subjects; and
(g) a comparison unit (170), configured to compare the value of the vasodilation parameter of the subject with the normative value.

11. The laser Doppler-based apparatus (100) of claim 10, further comprising an analysis unit (180), configured to evaluate the sensory nerve function and/or sympathetic activity of the subject based on the comparison result from the comparison unit (170).

12. The laser Doppler-based apparatus (100) of claim 11, wherein the output provided by the user interface (110) is at least one of, the vascular-related measurement, the vasodilation parameter, the comparison result from the comparison unit (170), and the analysis result from the analysis unit (180).

13. The laser Doppler-based apparatus (100) of claim 11, further comprising a storage unit, configured to store at least one additional vascular-related measurement measured at the same sampling site or at least one different sampling site of the subject, concurrently with or after the first sampling procedure.

14. The laser Doppler-based apparatus (100) of claim 13, wherein
the computing unit (150) is further configured to calculate an additional value of at least one vasodilation parameter from the at least one additional vascular-related measurement;
the comparison unit (170) is further configured to compare the additional value of the vasodilation parameter of the subject with the normative vasodilation parameter and/or with the vasodilation parameter; and
the analysis unit (180) is further configured to evaluate the sensory nerve function and/or sympathetic activity of the subject and/or the subject's response to an intervention based on the comparison result from the comparison unit (170).

15. The laser Doppler-based apparatus (100) of claim 10, wherein the laser Doppler detector (120) comprises a probe having a diameter of less than 5 mm.

**Patentansprüche**

1. Verfahren zur Erlangung eines Werts für wenigstens einen Vasodilatationsparameter, welcher das Ansprechen einer Testperson auf die kutane lokale thermische Hyperämie darstellt, unter Verwendung einer Vorrichtung auf Laser-Doppler-Basis (100), umfassend:

   (a) Durchführen einer Ausgangswertmessung an einer ersten Messstelle der Testperson bei einer ersten Temperatur und Erhalten eines ersten Ausgangswerts für den Fluss der roten Blutkörperchen ($RBCF_{BL}$) an der Messstelle und eines ersten Ausgangswerts für den mittleren arteriellen Druck ($P_{BL}$) der Testperson,
   (b) Erhöhen der Temperatur der ersten Messstelle von der ersten Temperatur auf eine zweite Temperatur,
   (c) Beibehalten der zweiten Temperatur für einen Anfangserwärmungszeitraum von 2 bis 14 Minuten und Aufzeichnung einer Mehrzahl von ersten Anfangs - RBCFs ($RBCF_{I, 1-n}$) an der ersten Messstelle zu einer Mehrzahl von Zeitpunkten ($T_{1-n}$), um ein erstes Anfangsmaximum des RBCF ($RBCF_{I, max}$) zu ermitteln, und Aufzeichnung eines ersten mittleren arteriellen Anfangsdrucks ($P_I$) der Testperson während des Anfangserwärmungszeitraums, und
   (d) Berechnen des Werts des Vasodilatationsparameters, wobei der Vasodilatationsparameter ein erstes Anfangsmaximum der kutanen vaskulären Leitfähigkeit ($CVC_{I, max}$) ist, welche durch die Formel: $CVC_{I, max}$ = $RBCF_{I, max} / P_I$ berechnet wird, oder von dieser abgeleitet ist, wobei der von der ersten $CVC_{I, max}$ abgeleitete Vasodilatationsparameter eine Änderung des ersten Anfangsmaximums der CVC ($\Delta CVC$) oder eine Anfangs-Fläche-unter-der-Kurve (Initial-AUC) ist, wobei $\Delta CVC$ durch die Formel: $\Delta CVC = CVC_{I, max} - (RBCF_{BL} / P_{BL})$ berechnet wird, und wobei für die Initial-AUC, $RBCF_{I, 1-n}$ gegen $T_{1-n}$ aufgetragen wird, um eine Kurve zu erhalten, die eine Kurvenfunktion F(X) hat, wobei die Initial-AUC durch die Formel: $AUC = \int_0^\tau F(X)dX$ berechnet wird, wobei t gleich der oder größer als die Zeit ist, zu welcher $RBCF_{I, max}$ gemessen wird.

2. Verfahren nach Anspruch 1, ferner umfassend:

   Ermitteln eines Wertes für ein zweites Anfangsmaximum der CVC an einer zweiten Messstelle der Testperson in einem Zeitabstand von 0,5 bis 2 Stunden durch Wiederholen der Schritte (a) bis (d).

3. Verfahren nach Anspruch 2, bei welchem die zweite Messstelle die gleiche wie die erste Messstelle ist.

4. Verfahren nach Anspruch 2, bei welchem die zweite Messstelle von der ersten Messstelle verschieden ist.

5. Verfahren nach Anspruch 4, bei welchem die erste Messstelle und die zweite Messstelle anatomisch symmetrische Punkte sind.

6. Verfahren nach Anspruch 1, bei welchem die Ausgangswertmessung für etwa 2 bis 5 Minuten durchgeführt wird.

7. Verfahren nach Anspruch 1, bei welchem der erste RBCF - Ausgangswert, die ersten Anfangs - RBCFs und das erste Anfangsmaximum des RBCF mit einer auf dem Laser-Doppler-Prinzip basierenden Messsonde, die einen Durchmesser von weniger als 5 mm hat, gemessen werden.

8. Verfahren nach Anspruch 1, wobei das Verfahren keinen nachfolgenden Erwärmungsschritt nach dem Anfangserwärmungszeitraum benötigt.

9. Verfahren nach einem der Ansprüche 1, bei weichem
   in dem Schritt (b) die Temperatur mit einer Heizrate von 0,02 - 0,2°C / Sekunde erhöht wird,
   die erste Temperatur etwa 28 - 35°C beträgt und
   die zweite Temperatur etwa 38 - 44°C beträgt.

10. Laser - Doppler - basierte Vorrichtung (100) zur Erlangung eines Werts für wenigstens einen Vasodilatationsparameter, welcher das Ansprechen einer Testperson auf die kutane lokale thermische Hyperämie darstellt, umfassend:

    (a) eine Benutzerschnittstelle (110), die zum Empfangen einer Eingabe von einem Benutzer und zur Bereitstellung eines Ergebnisses an den Benutzer konfiguriert ist,
    (b) einen Laser - Doppler - Detektor (120), der zur Durchführung von wenigstens einer die Blutgefäße betreffenden Messung an einer Messstelle der Testperson während einem ersten Abtastungsvorgang konfiguriert ist, bei welchem die die Blutgefäße betreffende Messung wahlweise ist: ein erster Ausgangswert für den Fluss der roten Blutkörperchen ($RBCF_{BL}$), ein erster Ausgangswert für den mittleren arteriellen Druck ($P_{BL}$), welcher während einer Ausgangswertmessung erhalten wird, ein erster Anfangs - RBCF ($RBCF_{I, 1-n}$), ein erstes Anfangsmaximum des RBCF ($RBCF_{I, max}$) und ein erster mittlerer arterieller Anfangsdruck ($P_I$), welcher während einem Anfangserwärmungszeitraum von 2 bis 14 Minuten erhalten wird,

(c) eine Heizeinheit (130), die zum Erwärmen der Messstelle konfiguriert ist,

(d) eine Steuerung (140), die zur Kontrolle des Laser - Doppler - Detektors (120) und der Heizeinheit basierend auf der Benutzereingabe oder einer Voreinstellung konfiguriert ist,

(e) eine Rechnereinheit (150), weiche zur Berechnung des Wertes des Vasodilatationsparameters aus der wenigstens einen die Blutgefäße betreffenden Messung konfiguriert ist, wobei der Vasodilatationsparameter ein erstes Anfangsmaximum der kutanen vaskulären Leitfähigkeit ($CVC_{I, max}$) ist, welche durch die Formel: $CVC_{I, max} = RBCF_{I, max} / P_t$ berechnet wird, oder von dieser abgeleitet ist, wobei der von der ersten $CVC_{I, max}$ abgeleitete Vasodilatationsparameter eine Änderung des ersten Anfangsmaximums der CVC ($\Delta CVC$) oder eine Anfangs-Fläche-unter-der-Kurve (Initial-AUC) ist, wobei $\Delta CVC$ durch die Formel: $\Delta CVC = CVC_{I, max} - (RBCF_{BL} / P_{BL})$ berechnet wird, und wobei für die Initial-AUC, $RBCF_{I, 1-n}$ gegen $T_{1-n}$ aufgetragen wird, um eine Kurve zu erhalten, die eine Kurvenfunktion F(X) hat, wobei die Initial-AUC durch die Formel: $AUC = \int_0^t F(X)dX$ berechnet wird, wobei t gleich der oder größer als die Zeit ist, zu welcher $RBCF_{I, max}$ gemessen wird,

(f) eine Datenbank (160), die einen normativen Wert für den Vasodilatationsparameter umfasst, welcher anhand einer Grundgesamtheit von gesunden Testpersonen ermittelt wurde, und

(g) eine Vergleichseinheit (170), die zum Vergleich des Wertes des Vasodilatationsparameters der Testperson mit dem normativen Wert konfiguriert ist.

**11.** Laser - Doppler - basierte Vorrichtung (100) nach Anspruch 10, welche ferner eine Analyseeinheit (180) umfasst, die zur Bewertung der sensorischen Nervenfunktion und/ oder Symphatikusaktivität der Testperson basierend auf dem Vergleichsergebnis von der Vergleichseinheit (170) konfiguriert ist.

**12.** Laser - Doppler - basierte Vorrichtung (100) nach Anspruch 11, bei welcher das Ergebnis, das durch die Benutzerschnittstelle (110) bereitgestellt wird, wahlweise wenigstens die die Blutgefäße betreffende Messung, der Vasodilatationsparameter, das Vergleichsergebnis aus der Vergleichseinheit (170) oder das Analyseergebnis aus der Analyseeinheit (180) ist.

**13.** Laser - Doppler - basierte Vorrichtung (100) nach Anspruch 11, welche ferner eine Speichereinheit umfasst, die konfiguriert ist, um wenigstens eine zusätzliche die Blutgefäße betreffende Messung zu speichern, die an der gleichen Messstelle oder wenigstens einer anderen Messstelle der Testperson gleichzeitig mit oder nach dem ersten Abtastungsvorgang gemessen wurde.

**14.** Laser - Doppler - basierte Vorrichtung (100) nach Anspruch 13, bei welcher die Rechnereinheit (150) ferner zur Berechnung eines zusätzlichen Wertes von wenigstens einem Vasodilatationsparameter aus der wenigstens einen zusätzlichen die Blutgefäße betreffenden Messung konfiguriert ist,
die Vergleichseinheit (170) ferner konfiguriert ist, um den zusätzlichen Wert für den Vasodilatationsparameter der Testperson mit dem normativen Vasodilatationsparameter und/ oder mit dem Vasodilatationsparameter zu vergleichen, und
die Analyseeinheit (180) ferner konfiguriert ist, um die sensorische Nervenfunktion und/ oder Symphatikusaktivität der Testperson und/ oder das Ansprechen der Testperson auf eine Maßnahme basierend auf dem Vergleichsergebnis von der Vergleichseinheit (170) zu bewerten.

**15.** Laser - Doppler - basierte Vorrichtung (100) nach Anspruch 10, bei welcher der Laser - Doppler - Detektor (120) eine Messsonde umfasst, die einen Durchmesser von weniger als 5 mm hat.

**Revendications**

**1.** Procédé d'obtention d'une valeur d'au moins un paramètre de vasodilatation représentant la réponse d'hyperémie thermique locale cutanée d'un sujet en utilisant un appareil basé sur Doppler laser (100), comprenant :

(a) l'exécution d'une mesure de ligne de base au niveau d'un premier site d'échantillonnage du sujet à une première température et l'obtention d'un premier flux de globules rouges de ligne de base ($RBCF_{BL}$) du site d'échantillonnage et une première pression artérielle moyenne de ligne de base ($P_{BL}$) du sujet;

(b) l'élévation de la température du premier site d'échantillonnage de la première température à une seconde température;

(c) le maintien de la seconde température pendant une période de chauffage initial de 2 à 14 minutes, et l'enregistrement d'une pluralité de premiers flux de globules rouges initiaux ($RBCF_{I, 1-n}$) du premier site d'échantillonnage à une pluralité de points temporels ($T_{1-n}$) pour identifier un premier flux de globules rouges maximal initial ($RBCF_{I, max}$) et l'enregistrement d'une première pression artérielle moyenne initiale ($P_I$) du sujet pendant la période de chauffage initial ; et

(d) le calcul de la valeur du paramètre de vasodilatation, dans lequel le paramètre de vasodilatation est ou est dérivé d'une première conductance vasculaire cutanée maximale initiale ($CVC_{I, max}$), calculée par la formule suivante : $CVC_{I, max} = RBCF_{I, max} / P_I$, et le paramètre de vasodilatation dérivé de la première $CVC_{I, max}$ est une première différence de CVC maximale initiale ($\Delta CVC$) ou une aire sous la courbe initiale (AUC initiale), dans lequel le $\Delta CVC$ est calculé par la formule suivante : $\Delta CVC = CVC_{I, max} - (RBCF_{BL} / P_{BL})$ ; et pour l'aire sous la courbe initiale, les $RBCF_{I, 1-n}$ sont tracés en fonction de $T_{1-n}$ pour obtenir une courbe possédant une fonction de courbe de F(X), et l'aire sous la courbe initiale est calculée par la formule suivante : $AUC = \int_0^t F(X)dX$, où t est égal ou supérieur au temps auquel le $RBCF_{I, max}$ est mesuré.

2. Procédé selon la revendication 1, comprenant en outre:

l'obtention d'une valeur d'une seconde CVC maximale initiale au niveau d'un second site d'échantillonnage du sujet à un intervalle temporel de 0,5 à 2 heures en répétant les étapes (a) à (d).

3. Procédé selon la revendication 2, dans lequel le second site d'échantillonnage est le même que le premier site d'échantillonnage.

4. Procédé selon la revendication 2, dans lequel le second site d'échantillonnage est différent du premier site d'échantillonnage.

5. Procédé selon la revendication 4, dans lequel le premier site d'échantillonnage et le second site d'échantillonnage sont des points anatomiquement symétriques.

6. Procédé selon la revendication 1, dans lequel la mesure de ligne de base est effectuée pendant environ 2 à 5 minutes.

7. Procédé selon la revendication 1, dans lequel le premier flux de globules rouges de ligne de base, les premiers flux de globules rouges initiaux et le premier flux de globules rouges maximal initial sont mesurés avec une sonde basée sur Doppler laser ayant un diamètre inférieur à 5 mm.

8. Procédé selon la revendication 1, où le procédé n'exige aucune étape de chauffage ultérieur après la période de chauffage initial.

9. Procédé selon l'une quelconque des revendications 1, dans lequel dans l'étape (b), la température est élevée à une vitesse de chauffage de 0,02 à 0,2 °C/s ;
la première température est d'environ 28 à 35 °C; et
la seconde température est d'environ 38 à 44 °C.

10. Appareil à base de Doppler laser (100) permettant d'obtenir une valeur d'au moins un paramètre de vasodilatation représentant la réponse d'hyperémie thermique locale cutanée d'un sujet, comprenant:

(a) une interface utilisateur (110), conçue pour recevoir une entrée d'un utilisateur et fournir une sortie à l'utilisateur;

(b) un détecteur de Doppler laser (120), conçu pour mesurer au moins une mesure vasculaire connexe au niveau d'un site d'échantillonnage du sujet pendant une première procédure d'échantillonnage, dans lequel la mesure vasculaire connexe est l'une quelconque parmi : un premier flux de globules rouges de ligne de base ($RBCF_{BL}$), une première pression artérielle moyenne de ligne de base ($P_{BL}$) obtenue pendant une mesure de ligne de base, un premier flux de globules rouges initial ($RBCF_{I, 1-n}$), un premier flux de globules rouges maximal initial ($RBCF_{I, max}$) et une première pression artérielle moyenne initiale ($P_I$) obtenue pendant une période de chauffage initial de 2 à 14 minutes;

(c) une unité de chauffage (130), conçue pour chauffer le site d'échantillonnage;

(d) un dispositif de commande (140), conçu pour commander le détecteur de Doppler laser (120) et l'unité de chauffage sur la base de l'entrée utilisateur ou d'un paramètre par défaut;

(e) une unité de calcul (150), conçue pour calculer la valeur du paramètre de vasodilatation à partir de ladite au moins une mesure vasculaire connexe, dans lequel le paramètre de vasodilatation est ou est dérivé d'une première conductance vasculaire cutanée maximale initiale ($CVC_{I, max}$) calculée par la formule suivante : $CVC_{I, max} = RBCF_{I, max} / P_I$, et le paramètre de vasodilatation dérivé de la première $CVC_{I, max}$ est une première différence de CVC maximale initiale ($\Delta CVC$) ou une aire sous la courbe initiale (AUC initiale), dans lequel le $\Delta CVC$ est calculé par la formule suivante : $\Delta CVC = CVC_{I, max} - (RBCF_{BL} / P_{BL})$ ; et pour l'aire sous la courbe initiale, les $RBCF_{I, 1-n}$ sont tracés en fonction de $T_{1-n}$ pour obtenir une courbe possédant une fonction de courbe de F(X), et l'aire sous la courbe initiale est calculée par la formule suivante : $AUC = \int_{0}^{t} F(X)dX$, où t est égal ou supérieur au temps auquel le $RBCF_{I, max}$ est mesuré;

(f) une base de données (160), comprenant une valeur normative du paramètre de vasodilatation obtenue auprès d'une population de sujets en bonne santé ; et

(g) une unité de comparaison (170), conçue pour comparer la valeur du paramètre de vasodilatation du sujet avec la valeur normative.

11. Appareil à base de Doppler laser (100) selon la revendication 10, comprenant en outre une unité d'analyse (180), conçue pour évaluer la fonction nerveuse sensorielle et/ou l'activité sympathique du sujet sur la base du résultat de comparaison provenant de l'unité de comparaison (170).

12. Appareil à base de Doppler laser (100) selon la revendication 11, dans lequel la sortie fournie par l'interface utilisateur (110) est au moins l'une parmi la mesure vasculaire connexe, le paramètre de vasodilatation, le résultat de comparaison provenant de l'unité de comparaison (170) et le résultat d'analyse provenant de l'unité d'analyse (180).

13. Appareil à base de Doppler laser (100) selon la revendication 11, comprenant en outre une unité de stockage, conçue pour stocker au moins une mesure vasculaire connexe supplémentaire mesurée au niveau du même site d'échantillonnage ou d'au moins un site d'échantillonnage différent du sujet, en même temps que, ou après, la première procédure d'échantillonnage.

14. Appareil à base de Doppler laser (100) selon la revendication 13, dans lequel l'unité de calcul (150) est en outre conçue pour calculer une valeur supplémentaire d'au moins un paramètre de vasodilatation à partir de ladite au moins une mesure vasculaire connexe supplémentaire;
l'unité de comparaison (170) est en outre conçue pour comparer la valeur supplémentaire du paramètre de vasodilatation du sujet avec le paramètre de vasodilatation normatif et/ou avec le paramètre de vasodilatation; et
l'unité d'analyse (180) est en outre configurée pour évaluer la fonction nerveuse sensorielle et/ou l'activité sympathique du sujet et/ou la réponse du sujet à une intervention sur la base du résultat de comparaison provenant de l'unité de comparaison (170).

15. Appareil à base de Doppler laser (100) selon la revendication 10, dans lequel le détecteur de Doppler laser (120) comprend une sonde ayant un diamètre inférieur à 5 mm.

100

110

140

130

120

200

200
| Computing unit | 150 |
| Database | 160 |
| Comparison unit | 170 |
| Analysis unit | 180 |
| Storage unit | 190 |

FIG. 1

FIG. 2

**EP 2 830 503 B1**

**REFERENCES CITED IN THE DESCRIPTION**

**Patent documents cited in the description**

- CN 1806757 A
- US 4859078 A
- US 2004067270 A1
- US 20110172749 A1